# EUROPEAN PATENT APPLICATION

(11) **EP 4 537 838 A1**
(43) Date of publication of application: **16.04.2025**
(21) Application number: 23820132.1
(22) Date of filing: 09.06.2023
(51) Int. Cl.: A61K 38/53, A61K 39/39, A61K 39/12, A61P 31/12, A61P 31/14, A23L 33/17, A61K 8/66, A61Q 19/00

(54) **NOVEL CRS FRAGMENT PEPTIDE WITH IMMUNOPOTENTIATING ACTIVITY, AND USE THEREOF**

(30) Priority: 09.06.2022 KR 20220070283; 09.06.2022 KR 20220070287
(71) Applicant: Zymedi Co., Ltd., Incheon 21983 (KR)
(72) Inventor: CHO, Seongmin, Yongin City Gyeonggi-do 16845 (KR); KIM, Sunghoon, Suwon-si Gyeonggi-do 16229 (KR)
(74) Representative: Hoefer & Partner Patentanwälte mbB
(86) International application number: PCT/KR2023/007930
(87) International publication number: WO 2023/239197

(57) **Abstract**

The present invention relates to a novel CRS fragment peptide with immune-enhancing activity and its uses, more specifically to a novel peptide consisting of the amino acid sequence of SEQ ID NO:2 or an amino acid sequence having 95% or more sequence homology thereto, and its use as a vaccine adjuvant, an anticancer agent, and an antiviral composition. The peptide disclosed in the present invention is a CRS fragment disclosed for the first time in this specification, exhibiting anticancer activity, immune function enhancement, and antiviral activity.

## Description

### TECHNICAL FIELD

This application claims priority to Korean Patent Application Nos. 10-2022-0070283 and 10-2022-0070287, filed on June 9, 2022, and the entire disclosures of which are incorporated herein by reference.

The present invention relates to novel CRS fragment peptides with immune-enhancing activity and uses thereof, and more specifically, to novel peptides consisting of the amino acid sequence of SEQ ID NO:2 or an amino acid sequence having 95% or more sequence homology thereto, and their uses as vaccine adjuvants, cancer therapeutics, and antiviral agents.

### BACKGROUND OF THE INVENTION

Aminoacyl-tRNA synthetase (ARS or AARS), which catalyzes the aminoacylation of tRNA molecules, is essential for decoding genetic information during the translation process. Each eukaryotic tRNA synthetase consists of a core enzyme (closely related to the prokaryotic counterpart of tRNA synthetase) and additional domains (attached to the amino or carboxy terminus of the core enzyme). Therefore, there are significant differences in the composition of these enzymes between eukaryotes and prokaryotes. For example, human tyrosyl-tRNA synthetase (TyrRS) has a carboxy-terminal domain that is absent in the TyrRS molecules of prokaryotes and lower eukaryotes.

Recently, several aminoacyl-tRNA synthetases have been shown to have non-canonical functions that are separate from their involvement in the translation process. Specifically, some fragments of ARS proteins exhibit extracellular signaling activity that regulates different types of pathways beyond protein translation, showing unexpected activities unrelated to aminoacylation. These unexpected activities can sometimes be therapeutically useful for certain diseases, but they may also pose a risk of inducing pathological conditions in humans. For instance, lysyl-tRNA synthetase (KRS) is known to have an activity that promotes cancer metastasis. Additionally, the N-terminal domain of TRS, known as mini-tyrosyl tRNA synthetase (mini-TRS, corresponding to amino acid residues 1-364), which is cleaved by polymorphonuclear cell elastase and plasmin, exhibits non-canonical biological activities not found in the full-length protein. In vitro, mini-TRS has been shown to stimulate endothelial cell proliferation and migration and has pro-angiogenic activity in mouse matrigel assays. The function of promoting angiogenesis is generally closely associated with cancer metastasis.

Such unexpected activities are not observed in the natural full-length protein sequence (or show insignificant effects at the natural full-length protein level) but may exhibit significant specific activities when certain regions are isolated. Additionally, these effects may have characteristics that are unsuitable for therapeutic use. To overcome the difficulties associated with this unpredictability and to harness the therapeutic potential of these ARS family proteins, various efforts are needed to identify biologically relevant forms of other aminoacyl-tRNA synthetase proteins.

Moreover, while vaccination is currently the best method for preventing viral diseases, the efficiency of vaccines is a significant issue due to the generation of many viral serotypes (subtypes) in the case of viral diseases. There is a need to develop antiviral compositions that can complement these vaccine issues, such as compositions for preventing or treating viral infections or adjuvants that can enhance the effectiveness of viral vaccines.

In particular, in January 2020, SARS-CoV-2, identified as the cause of COVID-19, spread worldwide and was declared a pandemic by the World Health Organization (WHO). The new coronavirus has been reported to be highly contagious and pathogenic, causing severe pneumonia symptoms in infected patients. This virus has been found to pose a serious threat, especially to the elderly and/or individuals with underlying conditions such as cardiovascular disease, diabetes, chronic respiratory disease, and cancer. However, the development of formulations that can effectively prevent and treat infections by new viruses like SARS-CoV-2 has not yet been completed.

### SUMMARY OF INVENTION

### PROBLEM TO BE SOLVED

The inventors of the present invention previously discovered that a fragment peptide of cysteinyl-tRNA synthetase (hereinafter referred to as "CRS") exhibits anticancer activity and immune-enhancing activity. However, the inventors confirmed that it is difficult to develop it as a drug due to the aforementioned limitations. Therefore, the inventors conducted extensive research to develop a novel CRS fragment peptide that can overcome these limitations. As a result, the inventors discovered that a peptide comprising consecutive amino acids from any amino acid selected from the 101st to the 140th amino acids to the 200th amino acid in the amino acid sequence of SEQ ID NO: 1 exhibits equivalent anticancer activity and immune-enhancing activity compared to the conventional CRS fragment peptide, while maintaining its form as a monomer, not degrading without an affinity tag, and being highly stable even at high temperatures, thereby completing the present invention.

Therefore, another object of the present invention is to provide a pharmaceutical composition, a food composition, or a quasi-drug composition for antiviral use comprising a peptide comprising consecutive amino acids from any amino acid selected from the 99th to the 140th amino acids to any amino acid selected from the 185th to the 228th amino acids in the amino acid sequence of SEQ ID NO: 1; a peptide comprising an amino acid sequence having 80% or more homology with the peptide; or a pharmaceutically acceptable salt thereof as an active ingredient.

Another object of the present invention is to provide a pharmaceutical composition, a food composition, or a quasi-drug composition for antiviral use consisting of a peptide comprising consecutive amino acids from any amino acid selected from the 99th to the 140th amino acids to any amino acid selected from the 185th to the 228th amino acids in the amino acid sequence of SEQ ID NO:1; a peptide comprising an amino acid sequence having 80% or more homology with the peptide; or a pharmaceutically acceptable salt thereof.

Another object of the present invention is to provide a pharmaceutical composition, a food composition, or a quasi-drug composition for antiviral use consisting essentially of a peptide comprising consecutive amino acids from any amino acid selected from the 99th to the 140th amino acids to any amino acid selected from the 185th to the 228th amino acids in the amino acid sequence of SEQ ID NO:1; a peptide comprising an amino acid sequence having 80% or more homology with the peptide; or a pharmaceutically acceptable salt thereof.

Another object of the present invention is to provide an adjuvant composition for a viral vaccine comprising a peptide comprising consecutive amino acids from any amino acid selected from the 99th to the 140th amino acids to any amino acid selected from the 185th to the 228th amino acids in the amino acid sequence of SEQ ID NO: 1; a peptide comprising an amino acid sequence having 80% or more homology with the peptide; or a pharmaceutically acceptable salt thereof as an active ingredient.

Another object of the present invention is to provide the use of a peptide comprising consecutive amino acids from any amino acid selected from the 99th to the 140th amino acids to any amino acid selected from the 185th to the 228th amino acids in the amino acid sequence of SEQ ID NO: 1; a peptide comprising an amino acid sequence having 80% or more homology with the peptide; or a pharmaceutically acceptable salt thereof for manufacturing a pharmaceutical composition for antiviral use.

Another object of the present invention is to provide a method for preventing or treating a viral infection, comprising administering to a subject in need thereof an effective amount of a composition comprising a peptide comprising consecutive amino acids from any amino acid selected from the 99th to the 140th amino acids to any amino acid selected from the 185th to the 228th amino acids in the amino acid sequence of SEQ ID NO: 1; or a peptide comprising an amino acid sequence having 80% or more homology with the peptide as an active ingredient.

An object of the present invention is to provide a peptide consisting of the amino acid sequence of SEQ ID NO:2 or an amino acid sequence having 95% or more sequence homology thereto.

Another object of the present invention is to provide a polynucleotide comprising a nucleotide sequence encoding the peptide.

Another object of the present invention is to provide a vector comprising the polynucleotide.

Another object of the present invention is to provide a host cell transformed with the vector.

Another object of the present invention is to provide a vaccine adjuvant comprising one or more selected from the group consisting of:
(i) the peptide,
(ii) a polynucleotide encoding (i),
(iii) a vector comprising (ii), and
(iv) a host cell transformed with (iii).

Another object of the present invention is to provide a vaccine composition comprising the vaccine adjuvant and an antigen.

Another object of the present invention is to provide a pharmaceutical composition for preventing or treating cancer comprising one or more selected from the group consisting of:
Another object of the present invention is to provide a pharmaceutical composition for preventing or treating cancer consisting of one or more selected from the group consisting of:
Another object of the present invention is to provide a pharmaceutical composition for preventing or treating cancer consisting essentially of one or more selected from the group consisting of:
(i) the peptide,
(ii) a polynucleotide encoding (i),
(iii) a vector comprising (ii), and
(iv) a host cell transformed with (iii).

### MEANS FOR SOLVING THE PROBLEM

To achieve the aforementioned objectives of the present invention, the present invention provides a pharmaceutical composition, food composition, or quasi-drug composition for antiviral use, comprising a peptide comprising consecutive amino acids sequence which is from any amino acid selected from 99th to 140th amino acids to any amino acid selected from 185th to 228th amino acids in the amino acid sequence of SEQ ID NO: 1; a peptide comprising an amino acid sequence having 80% or more homology with the peptide; or a pharmaceutically acceptable salt thereof as an active ingredient.

Additionally, to achieve the objectives of the present invention, the present invention provides a pharmaceutical composition, food composition, or quasi-drug composition for antiviral use, consisting of a peptide comprising consecutive amino acids sequence which is from any amino acid selected from 99th to 140th amino acids to any amino acid selected from 185th to 228th amino acids in the amino acid sequence of SEQ ID NO: 1; a peptide comprising an amino acid sequence having 80% or more homology with the peptide; or a pharmaceutically acceptable salt thereof.

Furthermore, to achieve the objectives of the present invention, the present invention provides a pharmaceutical composition, food composition, or quasi-drug composition for antiviral use, consisting essentially of a peptide comprising consecutive amino acids sequence which is from any amino acid selected from 99th to 140th amino acids to any amino acid selected from 185th to 228th amino acids in the amino acid sequence of SEQ ID NO:1; a peptide comprising an amino acid sequence having 80% or more homology with the peptide; or a pharmaceutically acceptable salt thereof.

To achieve another objective of the present invention, the present invention provides an adjuvant composition for a viral vaccine, comprising a peptide comprising consecutive amino acids sequence which is from any amino acid selected from 99th to 140th amino acids to any amino acid selected from 185th to 228th amino acids in the amino acid sequence of SEQ ID NO: 1; a peptide comprising an amino acid sequence having 80% or more homology with the peptide; or a pharmaceutically acceptable salt thereof as an active ingredient.

To achieve another objective of the present invention, the present invention provides the use of a peptide comprising consecutive amino acids sequence which is from any amino acid selected from 99th to 140th amino acids to any amino acid selected from 185th to 228th amino acids in the amino acid sequence of SEQ ID NO: 1; a peptide comprising an amino acid sequence having 80% or more homology with the peptide; or a pharmaceutically acceptable salt thereof for manufacturing a pharmaceutical composition for antiviral use.

To achieve another objective of the present invention, the present invention provides a method for preventing or treating a viral infection, comprising administering to a subject in need thereof an effective amount of a composition comprising a peptide comprising consecutive amino acids sequence which is from any amino acid selected from 99th to 140th amino acids to any amino acid selected from 185th to 228th amino acids in the amino acid sequence of SEQ ID NO:1; or a peptide comprising an amino acid sequence having 80% or more homology with the peptide as an active ingredient.

To achieve another objective of the present invention, the present invention provides a peptide consisting of the amino acid sequence of SEQ ID NO:2 or an amino acid sequence having 95% or more sequence homology thereto.

To achieve another objective of the present invention, the present invention provides a polynucleotide comprising a nucleotide sequence encoding the peptide.

To achieve another objective of the present invention, the present invention provides a vector comprising the polynucleotide.

To achieve another objective of the present invention, the present invention provides a host cell transformed with the vector.

To achieve another objective of the present invention, the present invention provides a vaccine adjuvant comprising one or more selected from the group consisting of:
(i) the peptide,
(ii) a polynucleotide encoding (i),
(iii) a vector comprising (ii), and
(iv) a host cell transformed with (iii).

To achieve another objective of the present invention, the present invention provides a vaccine composition comprising the vaccine adjuvant and an antigen.

To achieve another objective of the present invention, the present invention provides a pharmaceutical composition for preventing or treating cancer, comprising one or more selected from the group consisting of:
Additionally, to achieve another objective of the present invention, the present invention provides a pharmaceutical composition for preventing or treating cancer, consisting of one or more selected from the group consisting of:
Furthermore, to achieve another objective of the present invention, the present invention provides a pharmaceutical composition for preventing or treating cancer, consisting essentially of one or more selected from the group consisting of:
(i) the peptide,
(ii) a polynucleotide encoding (i),
(iii) a vector comprising (ii), and
(iv) a host cell transformed with (iii).

Hereinafter, the present invention will be described in detail.

The practice of the present invention, unless otherwise indicated, uses conventional methods of molecular biology and recombinant DNA technology within the technical field to which the present invention belongs, and most of the descriptions are well known for illustrative purposes.

All publications, patents, and patent applications cited herein are incorporated by reference in their entirety.

Throughout the disclosure, various aspects or conditions related to the present invention may be proposed in the form of ranges. Unless otherwise stated, the description of range values includes the boundary values (inclusive), meaning that it includes all values from the lower limit to the upper limit. The description of ranges is for convenience and brevity and should not be interpreted as an inflexible limitation on the scope of the present invention. Therefore, the description of ranges should be considered as specifically disclosing not only individual numerical values within the range but also all possible subranges. For example, the description of a range such as 7 to 170 should be considered as specifically disclosing individual values within the range, such as 9, 27, 35, 101, and 155, as well as subranges such as 10 to 127, 23 to 35, 80 to 100, and 50 to 169. This applies regardless of the width of the range.

The term "comprising" as used in the present specification is used interchangeably with "including" or "characterized by" and does not exclude additional components, elements, or method steps not mentioned in the composition or method. The term "consisting of" means excluding any additional elements, steps, or components not specifically mentioned. The term "consisting essentially of" means that the composition or method may include additional elements or steps that do not materially affect the basic characteristics of the composition or method.

The terms "peptide" and "protein" as used herein are used in their conventional sense, meaning an arrangement of amino acids. While peptides are not limited to a specific length, in the context of the present invention, they generally refer to fragments of full-length proteins and may include post-translational modifications such as glycosylation, acetylation, phosphorylation, and other modifications known in the field (both naturally occurring and non-naturally occurring modifications) and may be referred to as "polypeptides." The peptides and proteins of the present invention can be produced using any of various known recombinant and/or synthetic techniques, and exemplary embodiments will be further described below.

The present invention derives from the discovery that CRS and CRS-derived peptides possess therapeutically relevant non-canonical biological activities.

In this specification, 'non-canonical activity' generally refers to the activity possessed by the CRS peptide of the present invention, in addition to adding cysteine to tRNA molecules. As detailed herein, in certain embodiments, the non-canonical biological activities exhibited by the CRS fragment of the present invention can be selected from the group consisting of, but not limited to, anticancer activity, activation of innate immunity, and activation of adaptive immunity.

The present invention should be understood to include not only CRS fragment peptides having at least one non-canonical biological activity but also variants that substantially retain the non-canonical activity.

Specifically, the inventors have identified that novel CRS fragment peptides exhibit activity in activating both innate and adaptive immunity and are structurally very stable. The region of the CRS fragment peptide is disclosed for the first time in the present invention.

In the present invention, the peptide is a cleavage form of the CRS protein, comprising amino acids from the 140th to the 200th in the full-length CRS protein sequence of SEQ ID NO: 1, or amino acids having 95% or more sequence homology thereto, and retaining at least one desired non-canonical biological activity.

The present invention provides a food composition for antiviral use, comprising a peptide comprising consecutive amino acids sequence which is from any amino acid selected from the 99th to the 140th amino acids to any amino acid selected from the 185th to the 228th amino acids in the amino acid sequence of SEQ ID NO: 1; a peptide comprising an amino acid sequence having 80% or more homology with the peptide; or a pharmaceutically acceptable salt thereof as an active ingredient.

Additionally, the present invention provides a food composition for antiviral use, consisting of a peptide comprising consecutive amino acids sequence which is from any amino acid selected from the 99th to the 140th amino acids to any amino acid selected from the 185th to the 228th amino acids in the amino acid sequence of SEQ ID NO: 1; a peptide comprising an amino acid sequence having 80% or more homology with the peptide; or a pharmaceutically acceptable salt thereof.

Furthermore, the present invention provides a food composition for antiviral use, consisting essentially of a peptide comprising consecutive amino acids sequence which is from any amino acid selected from the 99th to the 140th amino acids to any amino acid selected from the 185th to the 228th amino acids in the amino acid sequence of SEQ ID NO: 1; a peptide comprising an amino acid sequence having 80% or more homology with the peptide; or a pharmaceutically acceptable salt thereof as an essential ingredient.

The description of each component in the food composition can refer to the aforementioned details.

The food composition of the present invention includes all types of food in the conventional sense, including functional foods, nutritional supplements, health foods, and food additives. These types of food compositions can be manufactured in various forms according to conventional methods known in the art.

The food composition of the present invention may include health functional foods. The term "health functional food" as used in the present invention refers to food manufactured and processed in the form of tablets, capsules, powders, granules, liquids, and pills using raw materials or ingredients with beneficial functionality for the human body. Here, 'functionality' means obtaining useful effects for health purposes such as regulating nutrients or physiological actions concerning the structure and function of the human body. The health functional food of the present invention can be manufactured by methods commonly used in the art, and during the manufacturing process, raw materials and ingredients commonly added in the art can be included.

Moreover, the formulation of the health functional food can be manufactured without limitation as long as it is recognized as a health functional food. The food composition of the present invention can be manufactured in various forms of formulations, and unlike general medicines, it has the advantage of not having side effects that may occur with long-term use of medicines since it uses food as a raw material. It is highly portable, and the health functional food of the present invention can be consumed as an adjuvant to enhance the improvement or treatment effect.

For example, health functional foods can be consumed in the form of tea, juice, and drinks, or in granulated, encapsulated, and powdered forms. Additionally, it can be manufactured in the form of a composition by mixing with known substances or active ingredients known to have preventive, improvement, or treatment effects.

Furthermore, the food composition of the present invention may contain various nutrients, vitamins, electrolytes, flavoring agents, coloring agents, pectic acid and its salts, alginic acid and its salts, organic acids, protective colloid thickeners, pH adjusters, stabilizers, preservatives, glycerin, alcohol, carbonating agents used in carbonated beverages, etc. It may also contain pulp for the manufacture of natural fruit juice, fruit juice beverages, and vegetable beverages. These ingredients can be used independently or in combination. The proportion of these additives is not particularly important, but it is generally selected in the range of 0.01 to 0.3 parts by weight per 100 parts by weight of the food composition of the present invention, but it is not limited thereto.

Additionally, the food composition of the present invention may contain various flavoring agents or natural carbohydrates as additional components, like conventional beverages. The carbohydrates include monosaccharides such as glucose and fructose, disaccharides such as maltose and sucrose, polysaccharides such as dextrin and cyclodextrin, and sugar alcohols such as xylitol, sorbitol, and erythritol. Natural sweeteners such as thaumatin and stevia extract, or synthetic sweeteners such as saccharin and aspartame can be used as sweeteners. The proportion of the natural carbohydrates is generally about 0.01 to 0.04 g, preferably about 0.02 to 0.03 g per 100 mL of the composition of the present invention, but it is not limited thereto.

The present invention also provides a quasi-drug composition for antiviral use, comprising a peptide comprising consecutive amino acids sequence which is from any amino acid selected from the 99th to the 140th amino acids to any amino acid selected from the 185th to the 228th amino acids in the amino acid sequence of SEQ ID NO: 1; a peptide comprising an amino acid sequence having 80% or more homology with the peptide; or a pharmaceutically acceptable salt thereof as an active ingredient.

In the "quasi-drug composition for antiviral use" according to the present invention, the quasi-drug is a preparation used for sterilization, insecticide, and similar purposes to prevent infectious diseases as described in subparagraph 7 of Article 2 of the Korea Pharmaceutical Affairs Act, and it can mean repellents, control agents, preventives, control agents, or attractant insecticides for flies, mosquitoes, etc., used for human or animal health.

Additionally, the quasi-drug may include external preparations for the skin and personal hygiene products. For example, it may be a sanitizing cleanser, nasal spray, shower foam, gargle, wet tissue, detergent soap, hand wash, humidifier filler, mask, ointment, patch, or filter filler, but it is not limited thereto.

When using the quasi-drug composition of the present invention as a quasi-drug additive, the composition can be added as it is or used together with other quasi-drugs or quasi-drug ingredients, and it can be appropriately used according to conventional methods. The mixing amount of the active ingredient can be suitably determined according to the intended use.

The quasi-drug composition of the present invention may be manufactured in the form of general emulsified formulations and solubilized formulations, for example. For instance, it may have formulations such as lotions, creams, ointments, sprays, oil gels, gels, oils, aerosols, and fumigants, but it is not limited to these as long as it exhibits the pest control inducing effect of the present invention. Additionally, the quasi-drug composition may appropriately be blended and used with oil, water, surfactants, moisturizers, lower alcohols with 1 to 4 carbon atoms, thickeners, chelating agents, pigments, preservatives, or fragrances, which are generally blended in quasi-drug compositions, as needed.

The present invention also provides an adjuvant composition for a viral vaccine, comprising a peptide comprising consecutive amino acids sequence which is from any amino acid selected from 99th to 140th amino acids to any amino acid selected from 185th to 228th amino acids in the amino acid sequence of SEQ ID NO: 1; a peptide comprising an amino acid sequence having 80% or more homology with the peptide; or a pharmaceutically acceptable salt thereof as an active ingredient.

In the present invention, the adjuvant can be defined as a substance that can present an antigen to the immune system in a manner that induces an immune response or an increase in the immune response to the antigen when the viral antigen is administered together with the adjuvant. That is, the adjuvant is a substance that enhances the immune response to the viral antigen, meaning a substance that is not an immunogen to the host but enhances immunity by increasing the activity of immune system cells.

According to one embodiment of the present invention, it has been confirmed that the CRS fragment peptide can significantly enhance the increase in the immune response of the subject when treated together with the viral antigen.

In one aspect of the present invention, the adjuvant can induce improved innate immune response and acquired immune response activation when administered together with the viral antigen compared to when the viral antigen itself is obtained, and more specifically, can induce improved cellular immune response and humoral immune response activation.

The present invention also provides the use of a peptide comprising consecutive amino acids sequence which is from any amino acid selected from 99th to 140th amino acids to any amino acid selected from 185th to 228th amino acids in the amino acid sequence of SEQ ID NO: 1; a peptide comprising an amino acid sequence having 80% or more homology with the peptide; or a pharmaceutically acceptable salt thereof for manufacturing a pharmaceutical composition for antiviral use.

The present invention also provides a method for preventing or treating a viral infection, comprising administering to a subject in need thereof an effective amount of a composition comprising a peptide comprising consecutive amino acids sequence which is from any amino acid selected from 99th to 140th amino acids to any amino acid selected from 185th to 228th amino acids in the amino acid sequence of SEQ ID NO:1; or a peptide comprising an amino acid sequence having 80% or more homology with the peptide as an active ingredient.

In one aspect of the present invention, the peptide is a peptide consisting of consecutive amino acids from any amino acid selected from 99th to 140th amino acids to any amino acid selected from 185th to 228th amino acids in the amino acid sequence of SEQ ID NO:1, and comprises a mutation in which the cysteine at position 182 in the amino acid sequence of SEQ ID NO:1 is substituted with another amino acid. That is, it may include a mutation in which the cysteine at position 43 in the amino acid sequence of SEQ ID NO:2 is substituted with another amino acid, and this substitution can not only inhibit the formation of multimers by the CRS fragment peptide but also maintain high stability without degradation even without affinity tags attached to the N-terminus and C-terminus.

In a preferred aspect of the present invention, the peptide is characterized in that the cysteine at position 182 in the amino acid sequence of SEQ ID NO:1 is substituted with serine. That is, it may include a mutation in which the cysteine at position 43 in the amino acid sequence of SEQ ID NO:2 is substituted with another amino acid (SEQ ID NO:3). Most preferably, the peptide is characterized by consisting of the amino acid sequences of SEQ ID NOs:2 to 8, or an amino acid sequence having 80% or more homology thereto.

The peptide of the present invention can be manufactured using available techniques known in the art. For example, it can be manufactured using various proteolytic enzymes. Exemplary proteases include, for instance, achromopeptidase, aminopeptidase, ancrod, angiotensin converting enzyme, bromelain, calpain, calpain I, calpain II, carboxypeptidase A, carboxypeptidase B, carboxypeptidase G, carboxypeptidase P, carboxypeptidase W, carboxypeptidase Y, caspase 1, caspase 2, caspase 3, caspase 4, caspase 5, caspase 6, caspase 7, caspase 8, caspase 9, caspase 10, caspase 11, caspase 12, caspase 13, cathepsin B, cathepsin C, cathepsin D, cathepsin E, cathepsin G, cathepsin H, cathepsin L, chymopapain, chymase, chymotrypsin, clostripain, collagenase, complement C1r, complement C1s, complement Factor D, complement factor I, cucumisin, dipeptidyl peptidase IV, elastase, leukocyte, elastase, pancreatic, endoproteinase Arg-C, endoproteinase Asp-N, endoproteinase Glu-C, endoproteinase Lys-C, enterokinase, factor Xa, ficin, furin, granzyme A, granzyme B, HIV Protease, IGase, kallikrein tissue, leucine aminopeptidase, general, leucine aminopeptidase, cytosol, leucine aminopeptidase, microsomal, matrix metalloprotease, methionine aminopeptidase, neutrase, papain, pepsin, plasmin, prolidase, pronase E, prostate specific antigen, protease alkalophilic from Streptomyces griseus, protease from Aspergillus, protease from Aspergillus saitoi, protease from Aspergillus sojae, protease B. licheniformis, alkaline or alcalase, protease from Bacillus polymyxa, protease from Bacillus sp, protease from Rhizopus sp., protease S, proteasomes, proteinase from Aspergillus oryzae, proteinase 3, proteinase A, proteinase K, protein C, pyroglutamate aminopeptidase, rennin, streptokinase, subtilisin, thermolysin, thrombin, tissue plasminogen activator, trypsin, tryptase, and urokinase. A person skilled in the art can easily determine which proteolytic enzyme is appropriate considering the chemical specificity of the fragment to be produced.

The polypeptides described herein can be manufactured by any suitable procedure known to those skilled in the art, such as recombinant techniques. In addition to recombinant production methods, the polypeptides of the present invention can be manufactured by direct peptide synthesis using solid-phase techniques.

Solid-phase peptide synthesis (SPPS) can initiate synthesis by attaching functional units called linkers to small porous beads, allowing the peptide chain to extend. Unlike liquid-phase methods, the peptide is covalently bonded to the bead, preventing it from being washed away during filtration until it is cleaved by specific reagents like trifluoroacetic acid (TFA). The synthesis involves cycles of protection, deprotection, and coupling processes, where the N-terminal amine of the attached peptide binds with an N-protected amino acid unit, followed by deprotection and coupling with a new amino acid. This SPPS method can be performed using microwave technology, which applies heat to shorten the time required for coupling and deprotection in each cycle. The heat energy helps prevent the expanding peptide chain from folding or aggregating and promotes chemical bonding.

Additionally, the peptides of the present invention can be synthesized by known methods using liquid-phase peptide synthesis. The peptides of the present invention can be synthesized by various methods, including a combination of the solid-phase and liquid-phase synthesis methods, and the manufacturing method is not limited to the means described herein.

Protein synthesis can be performed manually or through automation. Automated synthesis can be achieved using, for example, an Applied Biosystems 431A peptide synthesizer (Perkin Elmer). Alternatively, various fragments can be chemically synthesized separately and combined using chemical methods to produce the target molecule.

The peptides provided by the present invention include variants having 95% or more sequence homology with the peptide. These variants refer to active variants of the peptide, meaning they retain at least one desired irregular activity (e.g., anticancer activity and immune-enhancing activity) from the peptide they are derived from. An example of such a variant could be a splice variant, whether naturally or non-naturally occurring, which retains at least one irregular activity described herein. Another example could be a variant with one or more point mutations in the peptide sequence, whether naturally or non-naturally occurring, retaining at least one irregular activity described herein. In other words, the term 'active variant' in the present invention is understood as a functional equivalent of the peptide of SEQ ID NO:2.

More specifically, the variant is characterized by being a functional equivalent with at least 80%, 81%, 82%, 83%, 84%, 85%, 86%, 87%, 88%, 89%, 90%, 91%, 92%, 93%, 94%, 95%, 96%, 97%, 98%, or 99% sequence homology along its length to the aforementioned peptide sequence.

In one aspect of the present invention, the peptide may consist of the amino acid sequence of SEQ ID NO:2 or SEQ ID NO:3.

The variant may include any modification in the amino acid sequence of the 'peptide,' including one or more substitutions, deletions, additions, and/or insertions. Such variants can occur naturally or be synthetically produced by modifying or altering one or more of the peptide sequences of the present invention and evaluating their biological activity as described herein using any of a number of techniques well known in the art.

In one embodiment of the present invention, the variant includes conservative substitutions. 'Conservative substitution' refers to a substitution where one amino acid is replaced with another amino acid having similar properties, such that a person skilled in the art would predict that the secondary structure and hydropathic nature (hydrophobic or hydrophilic properties) of the peptide remain substantially unchanged. Generally, the following groups of amino acids exhibit conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Modifications can be made within the structure of the peptide of the present invention to obtain functional molecules encoding peptide variants or derivatives with desired characteristics. To create equivalent or improved variants of the peptide of the present invention, one or more codons can be altered based on protein codon information known in the art.

For example, specific amino acids can be substituted within the protein or peptide structure without significant loss of interactive binding ability, such as receptor binding sites, antigen-binding sites of antibodies, or substrate molecules. This is due to the interactive capabilities and properties of the protein, which define its biological functional activity, allowing specific amino acid sequence substitutions within the protein or peptide sequence and the underlying DNA coding sequence while still obtaining a protein with the same or similar properties.

Therefore, various changes in the disclosed peptide sequence or the DNA sequence encoding the peptide are considered without significant loss of desired utility or activity. These modifications may also consider the hydropathic index of amino acids. The importance of the hydropathic amino acid index, which imparts interactive biological functions to proteins, is generally understood in the art. For example, the relative hydropathic properties of amino acids contribute to the secondary structure of the resulting protein, ultimately defining its interactions with other molecules, such as enzymes, substrates, receptors, DNA, antibodies, antigens, etc. Each amino acid is assigned a hydropathic index based on its hydrophobic and charge characteristics. These values are as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamic acid (-3.5); glutamine (-3.5); aspartic acid (-3.5); asparagine (-3.5); lysine (-3.9); and arginine (-4.5).

It is known in the art that specific amino acids can be substituted with other amino acids having similar hydropathic indices or scores, resulting in proteins with similar biological activities (i.e., still obtaining biologically functionally equivalent proteins). In such changes, amino acids with a hydropathic index within ±2 are preferred for substitution, those within ±1 are particularly preferred, and those within ±0.5 are even more particularly preferred.

It is also understood in the art that the substitution of the same amino acid can be effectively performed based on hydrophilicity. As is known, the following hydrophilicity values are assigned to amino acid residues: Arginine (+3.0); Lysine (+3.0); Aspartic acid (+3.0±1); Glutamic acid (+3.0±1); Serine (+0.3); Asparagine (+0.2); Glutamine (+0.2); Glycine (0); Threonine (-0.4); Proline (-0.5±1); Alanine (-0.5); Histidine (-0.5); Cysteine (-1.0); Methionine (-1.3); Valine (-1.5); Leucine (-1.8); Isoleucine (-1.8); Tyrosine (-2.3); Phenylalanine (-2.5); Tryptophan (-3.4). It is understood that an amino acid can be substituted with another amino acid having a similar hydrophilicity value, and a biologically equivalent protein can be obtained. In such modifications, substitution of amino acids with a hydrophilicity value within ±2 is preferred, substitution of amino acids with a hydrophilicity value within ±1 is particularly preferred, and substitution of amino acids with a hydrophilicity value within ±0.5 is more particularly preferred.

As outlined above, amino acid substitution can be based on the relative similarity of the side-chain substituents of the amino acids, for example, based on their hydrophobicity, hydrophilicity, charge, size, etc. Exemplary substitutions considering the various features described above are well known to those skilled in the art and include the following: Arginine and Lysine; Glutamic acid and Aspartic acid; Serine and Threonine; Glutamine and Asparagine; Valine, Leucine, and Isoleucine.

Amino acid substitution can also be performed based on the similarity of the polarity, charge, solubility, hydrophobicity, hydrophilicity, and/or amphipathic properties of the residues. For example, negatively charged amino acids include Aspartic acid and Glutamic acid; positively charged amino acids include Lysine and Arginine; and amino acids with non-charged polar head groups having similar hydrophilicity values include Leucine, Isoleucine, and Valine; Glycine and Alanine; Asparagine and Glutamine; Serine, Threonine, Phenylalanine, and Tyrosine.

The variants may also include non-conservative changes. In a preferred embodiment, the variant peptide may differ from the natural sequence by substitution, deletion, or addition of five or fewer amino acids. Variants can also be modified by deletion or addition of amino acids that have minimal impact on the secondary structure and hydropathic properties of the peptide.

The peptide may include a signal (or leader) sequence at the N-terminus of the protein, which directs the transport of the protein either co-translationally or post-translationally. The peptide may also be conjugated with linker sequences or other sequences to facilitate synthesis, purification, or identification of the peptide (e.g., polyHis) or to enhance binding to a solid support. For example, the peptide may be conjugated to the Fc region of an immunoglobulin.

When comparing peptide sequences, as described below, two sequences are considered "identical" if the amino acid sequences in the two sequences are the same when the sequences are aligned for maximum correspondence. Comparison between two sequences is typically performed by identifying and comparing local regions of sequence similarity by comparing alignments within a comparison window. As used herein, a "comparison window" refers to a segment of at least about 20 contiguous positions, typically 30 to 75, or 40 to 50 contiguous positions, in which the sequences can be compared to a reference sequence after optimal alignment of the two sequences.

Optimal alignment of sequences for comparison can be performed using, for example, the Megalign program in the Lasergene suite of bioinformatics software (DNASTAR, Inc., Madison, Wis.) using default parameters. This program includes several alignment schemes described in the following references: Dayhoff, M. O. (1978) A model of evolutionary change in proteins-Matrices for detecting distant relationships. In Dayhoff, M. O. (ed.) Atlas of Protein Sequence and Structure, National Biomedical Research Foundation, Washington D.C. Vol. 5, Suppl. 3, pp. 345-358; Hein J. (1990) Unified Approach to Alignment and Phylogenes pp. 626-645 Methods in Enzymology vol. 183, Academic Press, Inc., San Diego, Calif.; Higgins, D. G. and Sharp, P. M. (1989) CABIOS 5:151-153; Myers, E. W. and Muller W. (1988) CABIOS 4:11-17; Robinson, E. D. (1971) Comb. Theor 11:105; Santou, N. Nes, M. (1987) Mol. Biol. Evol. 4:406-425; Sneath, P. H. A. and Sokal, R. R. (1973) Numerical Taxonomy-the Principles and Practice of Numerical Taxonomy, Freeman Press, San Francisco, Calif.; Wilbur, W. J. and Lipman, D. J. (1983) Proc. Nat'l Acad., Sci. USA 80:726-730.

Alternatively, optimal alignment of sequences for comparison can be performed using the partial identity algorithm of Smith and Waterman (1981) Add.APL.Math 2:482, the identity alignment algorithm of Needleman and Wunsch (1970) J.Mol.Biol.48:443, the similarity search method of Pearson and Lipman (1988) Proc.Natl.Acad.Sci.USA 85:2444 or the computerized implementations of these algorithms (GAP, BESTFIT, BLAST, FASTA, and TFASTA in the Wisconsin Genetics Software Package, Genetics Computer Group (GCG), 575 Science Dr., Madison, Wis.), or by inspection.

Examples of suitable algorithms for determining sequence identity and sequence similarity percentages include the BLAST and BLAST2.0 algorithms, which are described in Altschul et al. (1977) Nucl. Acids Res.25:3389-3402 and Altschul et al. (1990) J.Mol.Biol.215:403-410, respectively. BLAST and BLAST2.0 can be used to determine the sequence homology percentage of the polynucleotides and polypeptides of the present invention, for example, using the parameters described herein. Software for performing BLAST analysis is publicly available through the National Center for Biotechnology Information. For amino acid sequences, a scoring matrix can be used to calculate the cumulative score.

The extension of word hits in each direction stops in the following cases: if the cumulative alignment score decreases by quantity X from its maximum achieved value, if the cumulative score becomes zero or less due to the alignment of one or more negatively scoring residues, or if the end of either sequence is reached. The BLAST algorithm parameters W, T, and X determine the sensitivity and speed of the alignment.

In one exemplary approach, the 'percentage of sequence homology' is determined by comparing two optimally aligned sequences within a comparison window of at least 20 positions, where the polypeptide sequence portion in the comparison window may include up to 20 percent, typically 5-15 percent, or 10-12 percent of additions or deletions (i.e., gaps) compared to the reference sequence (which does not include additions or deletions). The percentage is calculated by determining the number of positions with identical amino acid residues in both sequences, obtaining the number of matching positions, dividing the number of matching positions by the total number of positions in the reference sequence (i.e., window size), and multiplying the result by 100 to yield the percentage of sequence homology.

The peptides provided in the present invention can be in linear form or cyclic form, which can be understood with reference to the embodiments of the present invention.

In the present invention, the production of cyclic peptides as the variant is not particularly limited to specific cyclization methods and forms, as long as it is by known peptide cyclization methods in the art. Preferably, the production of the cyclic peptide of the present invention may be performed by preparing a linear peptide with cysteine positioned at both termini (N-terminus and C-terminus) through cutting or substitution, and then forming a monosulfide bond between the cysteine residues present at both termini.

The peptide provided in the present invention, in one aspect, considers the use of modified polypeptides, and such modified polypeptides include modifications to improve the desired properties of the isolated polypeptide as described herein. Exemplary modifications of the polypeptide of the present invention include, but are not limited to, chemical derivatization and/or enzymatic derivatization of one or more constituent amino acids, including side chain modifications, backbone modifications, and modifications of the N-terminus and C-terminus, such as acetylation, hydroxylation, methylation, amidation, and addition of carbohydrate or lipid components, and other moieties. An exemplary modification includes PEGylation of the polypeptide.

In certain aspects, chemoselective ligation techniques may be used to modify the peptide of the present invention, for example, by attaching polymers in a site-specific and controlled manner. These techniques typically rely on the covalent attachment of a chemoselective anchor into the protein backbone by chemical or recombinant means, followed by subsequent modification with a polymer carrying a complementary linker. As a result, the covalent structure of the assembly process and the obtained protein-polymer conjugate is controlled, allowing for rational optimization of drug properties such as efficacy and pharmacokinetic characteristics. For instance, allowing selective attachment of PEG improves their pharmacokinetic properties.

The peptide of the present invention may include pharmaceutically acceptable salts. Examples of such pharmaceutically acceptable salts include, but are not limited to, hydrochloride, sulfate, phosphate, acetate, citrate, tartrate, succinate, lactate, maleate, fumarate, oxalate, methanesulfonate, or p-toluenesulfonate.

The present invention also provides a polynucleotide encoding the peptide.

The terms "DNA," "polynucleotide," and "nucleic acid" as used in the specification refer to DNA molecules isolated from the total genomic DNA of a specific species. Therefore, a DNA fragment encoding a polypeptide refers to a DNA fragment substantially isolated from the total genomic DNA of the species from which the DNA fragment can be obtained, or a DNA fragment consisting of one or more purified coding sequences. The terms 'DNA fragment' and 'polynucleotide' include DNA fragments and smaller fragments thereof, as well as recombinant vectors (e.g., plasmids, cosmids, phagemids, bacteriophages, viruses, etc.).

As understood by those skilled in the art, the polynucleotide sequence of the present invention is modified to express or capable of expressing proteins, peptides, etc., including genomic sequences, extra-genomic sequences, sequences encoded in plasmids, and smaller manipulated gene fragments. These fragments can be naturally isolated or synthetically modified by human hands.

As recognized by those skilled in the art, polynucleotides can be single-stranded (coding sequence or antisense sequence) or double-stranded, and can be DNA molecules (genomic, cDNA, or synthetic) or RNA molecules. Additional coding or non-coding sequences may be present within the polynucleotide of the present invention. Furthermore, polynucleotides can be linked to other molecules and/or support materials.

Polynucleotides may include natural sequences, variants, or biologically functional equivalents of the sequences. Polypeptide variants may include one or more substitutions, additions, deletions, and/or insertions, as further described below, preferably performed in a manner that does not substantially reduce the desired activity of the encoded polypeptide compared to the unmodified polypeptide. The effect on the activity of the encoded polypeptide can generally be evaluated as described herein.

The polynucleotide provided in the present invention is not particularly limited in its specific sequence as long as it encodes the peptide or its variant peptide of the present invention, and any combination of nucleotide sequences (nucleic acid sequences) is allowed. For example, a peptide consisting of the amino acid sequence of SEQ ID NO:2 can be expressed by a polynucleotide comprising the nucleotide sequence represented by SEQ ID NO:9, and a peptide consisting of the amino acid sequence of SEQ ID NO:3 can be expressed by a polynucleotide comprising the nucleotide sequence represented by SEQ ID NO: 10, but is not limited thereto.

The polynucleotide of the present invention, regardless of the length of its coding sequence itself, can be combined with other DNA sequences such as promoters, polyadenylation signals, additional restriction enzyme sites, multiple cloning sites, other coding fragments, etc., resulting in a significantly variable overall length. Therefore, polynucleotide fragments of almost any length are considered applicable, preferably limited by the ease of manufacture and use in the intended recombinant DNA protocols.

Furthermore, it is apparent to those skilled in the art that many nucleotide sequences encoding the peptides described herein exist as a result of the degeneracy of the genetic code. Some of these polynucleotides have minimal homology to the nucleotide sequence of any native gene. Nevertheless, other polynucleotides (e.g., polynucleotides optimized for codon selection in humans and/or primates) are specifically considered by the present invention due to differences in codon usage.

Additionally, alleles of genes comprising the polynucleotide sequences provided herein are within the scope of the present invention. Alleles are endogenous genes modified as a result of one or more variations, such as deletions, additions, and/or substitutions of nucleotides. The resulting mRNA and protein may have altered structures or functions (though not necessarily). Alleles can be identified using standard techniques (e.g., hybridization, amplification, and/or database sequence comparison).

Polynucleotides and their fusions can be manufactured, manipulated, and/or expressed using any of the well-established techniques known and available in the art. For example, polynucleotide sequences encoding the peptide of the present invention or its functional equivalent can be used within recombinant DNA molecules directing the expression of the polypeptide in appropriate host cells. Due to the inherent degeneracy of the genetic code, other DNA sequences encoding substantially the same or functionally equivalent amino acid sequences can be generated and used for cloning and expressing a given polypeptide.

As understood by those skilled in the art, in some cases, it is advantageous to produce nucleotide sequences (encoding polypeptides) that contain codons not naturally occurring. For example, codons preferred in specific prokaryotic or eukaryotic hosts can be selected to produce recombinant RNA transcripts with increased protein expression rates or desired properties (e.g., longer half-life than transcripts produced from naturally occurring sequences).

The polynucleotide sequence of the present invention can be manipulated using methods generally known in the art to alter the peptide-encoding sequence for various reasons, including but not limited to cloning, processing, expression, and/or modification of the activity of the gene product.

The present invention also provides a vector comprising the polynucleotide and a host cell transformed with the vector.

To express the desired polypeptide, a nucleotide sequence encoding the polypeptide or its functional equivalent can be inserted into an appropriate expression vector (i.e., a vector comprising elements necessary for the transcription and translation of the inserted coding sequence). Using methods well known to those skilled in the art, an expression vector comprising a sequence encoding the polypeptide of interest, and appropriate transcriptional and translational control elements can be constructed. These methods include in vitro recombinant DNA techniques, synthetic techniques, and in vivo genetic recombination.

Various expression vector/host systems are known and can be used to contain and express the polynucleotide sequence. The expression vector/host systems include, but are not limited to, bacteria transformed with recombinant bacteriophage, plasmid, or cosmid DNA expression vectors; yeast transformed with yeast expression vectors; insect cell systems infected with viral expression vectors (e.g., baculovirus); plant cell systems transformed with viral expression vectors (e.g., cauliflower mosaic virus, CaMV; tobacco mosaic virus, TMV) or bacterial expression vectors (e.g., Ti or pBR322 plasmids); or animal cell systems.

"Control elements" or "regulatory sequences" present in the expression vector are untranslated regions (enhancers, promoters, 5' and 3' untranslated regions) that interact with host cell proteins to carry out transcription and translation. These elements can vary in their strength and specificity. Depending on the vector system and host used, appropriate transcriptional and translational elements (including constitutive promoters and inducible promoters) can be utilized.

For example, in bacterial systems, inducible promoters such as the hybrid lacZ promoter of the pBLUESCRIPT phagemid (Stratagene, La Jolla, Calif.) or the pSPORT1 plasmid (Gibco BRL, Gaithersburg, MD) can be used. In mammalian cell systems, promoters derived from mammalian genes or mammalian viruses are generally preferred. If it is necessary to generate cell lines containing multiple copies of the sequence encoding the polypeptide, SV40 or EBV-based vectors can be usefully employed with appropriate selection markers.

In bacterial systems, a number of expression vectors can be selected depending on the intended use for peptide expression. For example, if large quantities are required, vectors aimed at high-level expression of easily purified fusion proteins can be used. Such vectors include, but are not limited to, the following: multifunctional E. coli cloning and expression vectors, such as BLUESCRIPT (Stratagene), where the sequence encoding the desired polypeptide is linked in-frame within the vector to the sequence for the amino-terminal Met and subsequent seven residues of β-galactosidase, resulting in the production of a hybrid protein; pIN vectors (Van Heeke and Schuster, J. Biol. Chem. 264:5503-5509 (1989)); and others. pGEX vectors (Promega, Madison, Wis.) can also be used to express foreign polypeptides as fusion proteins with glutathione S-transferase (GST). Generally, such fusion proteins are soluble and can be easily purified from lysed cells by adsorption to glutathione-agarose beads and subsequent elution in the presence of free glutathione. Proteins produced in these systems can be designed to include cleavage sites for proteases such as heparin, thrombin, or Factor Xa to release the cloned polypeptide from the GST moiety.

In yeast (Saccharomyces cerevisiae), a number of vectors comprising constitutive or inducible promoters (e.g., alpha factor, alcohol oxidase, and PGH) can be used.

When using plant expression vectors, the expression of the sequence encoding the polypeptide can be driven by any number of promoters. For example, viral promoters (e.g., the 35S promoter and 19S promoter of CaMV) can be used alone or in combination with the ω leader sequence derived from TMV. Alternatively, plant promoters (e.g., the small subunit of RUBISCO or heat shock promoters) can be used. These constructs can be introduced into plant cells by direct DNA transformation or pathogen-mediated transfection. These techniques are well known in the art.

Insect systems can also be used to express the desired polypeptide. For example, in one system, AcNPV (Autographa californica nuclear polyhedrosis virus) is used as a vector to express foreign genes in Spodoptera frugiperda cells or Trichoplusia larvae. The sequence encoding the polypeptide can be cloned into a non-essential region of the virus, such as the polyhedrin gene, under the control of the polyhedrin promoter. Successful insertion of the sequence encoding the polypeptide inactivates the polyhedrin gene and produces a recombinant virus deficient in coat protein. The recombinant virus can then be used to infect, for example, S. frugiperda cells or Trichoplusia larvae, where the desired polypeptide can be expressed.

In mammalian host cells, a number of virus-based expression systems are generally available. For example, when adenovirus is used as an expression vector, the sequence encoding the desired polypeptide can be linked within the adenovirus transcription/translation complex comprising the late promoter and tripartite leader sequence. By inserting into the non-essential E1 or E3 region of the viral genome, a viable virus capable of expressing the polypeptide in infected host cells can be obtained. Additionally, transcriptional enhancers (e.g., Rous Sarcoma Virus (RSV) enhancer) can be used to increase expression in mammalian host cells.

Additionally, specific initiation signals can be used for more efficient translation of the sequence encoding the desired polypeptide. These signals include the ATG start codon and adjacent sequences. When the sequence encoding the polypeptide, its start codon, and upstream sequences are inserted into an appropriate expression vector, additional transcriptional or translational control signals may not be necessary. However, if only the coding sequence or a portion thereof is inserted, an exogenous translational control signal comprising the ATG start codon must be provided. The start codon must also be in the correct reading frame to ensure translation of the entire insert. Exogenous translational elements and start codons can be derived from various origins (both natural and synthetic). The efficiency of expression can be enhanced by including appropriate enhancers for the specific cell system used (e.g., as described in Scharf et al., Results Probl. Cell Differ. 20:125-162 (1994)).

Also, the host cell line can be selected based on its ability to regulate the expression of the inserted sequence or to process the expressed protein in a desired manner. Such modifications of the polypeptide include, but are not limited to, acetylation, carboxylation, glycosylation, phosphorylation, lipidation, and acylation. Post-translational processing that cleaves the 'prepro' form of the protein can be utilized to promote proper insertion, folding, and/or function. Other host cells (e.g., CHO, HeLa, MDCK, HEK293, and W138, which have specific cellular machinery and mechanisms for such post-translational activities) can be selected to ensure accurate modification and processing of the foreign protein.

In the long term, stable expression is generally preferred for high-yield production of recombinant proteins. For example, a cell line that stably expresses the desired polynucleotide can be transformed using an expression vector, which may include viral replication origins and/or endogenous expression elements, and a selectable marker gene on the same or a separate vector.

After transducing the vector, cells can be grown in enriched media for 1-2 days, followed by transfer to selective media. The purpose of the selectable marker is to confer resistance to selection, and its presence allows for the growth and recovery of cells successfully expressing the introduced sequence. Resistant clones of stably transformed cells can be propagated using tissue culture techniques appropriate for the cell type.

Transformed cell lines can be recovered using various selection systems. Such selection systems include, but are not limited to, the herpes simplex virus thymidine kinase gene (Wigler et al., Cell 11:223-232 (1977)) and the adenine phosphoribosyltransferase gene (Lowy et al., Cell 22:817-823 (1990)), which can be used in tk- cells or aprt- cells, respectively.

Additionally, resistance to antimetabolites, antibiotics, or herbicides can be used as a basis for selection. For example, dhfr confers resistance to methotrexate (Wigler et al., Proc. Natl. Acad. Sci. U.S.A. 77:3567-70 (1980)); npt confers resistance to aminoglycosides, neomycin, and G-418 (Colbere-Garapin et al., J. Mol. Biol. 150:1-14 (1981)); and als or pat confer resistance to chlorsulfuron and phosphinothricin acetyltransferase, respectively. Additional selectable genes are known, such as trpB, which allows cells to use indole instead of tryptophan, or hisD, which allows cells to use histinol instead of histidine. Visible markers like anthocyanin, β-glucuronidase and its substrate GUS, and luciferase and its substrate luciferin are gaining popularity and are widely used not only to identify transformants but also to quantify the amount of transient or stable protein expression due to specific vector systems.

Various protocols for detecting and measuring the expression of the product encoded by the polynucleotide using either polyclonal or monoclonal antibodies specific to the product are known in the art. Examples include ELISA (enzyme-linked immunosorbent assay), RIA (radioimmunoassay), and FACS (fluorescence-activated cell sorting). These assay methods and additional ones can be referenced in the following literature: Hampton et al., Serological Methods, a Laboratory Manual (1990) and Maddox et al., J. Exp. Med. 158:1211-1216 (1983). Various labeling and conjugation techniques known to those skilled in the art can be used in various nucleic acid and amino acid assays. Means for producing labeled hybridization probes or labeled PCR probes to detect sequences related to the polynucleotide include oligolabeling, nick translation, end labeling, or PCR amplification using labeled nucleotides. Alternatively, the sequence or any part thereof can be cloned into a vector for the production of mRNA probes. Such vectors are known and commercially available, and can be used to synthesize RNA probes in vitro by adding appropriate RNA polymerases (e.g., T7, T3, or SP6) and labeled nucleotides. These procedures can be performed using various commercially available kits. Suitable reporter molecules or labels include radionuclides, enzymes, fluorophores, chemiluminescent agents, chromogenic agents, substrates, ligands, inhibitors, magnetic particles, and others.

Host cells transformed with the desired polynucleotide sequence can be cultured under conditions suitable for the expression of the protein and its recovery from the cell culture. The protein produced by the recombinant cells may be secreted or contained within the cells, depending on the sequence and/or vector.

As understood by those skilled in the art, expression vectors comprising the polynucleotide of the present invention can be designed to include signal sequences that direct the secretion of the polypeptide through prokaryotic or eukaryotic cell membranes. Other recombinant constructs can be used to link the sequence encoding the desired polypeptide to sequences encoding polypeptide domains that facilitate the purification of soluble proteins.

In addition to recombinant production methods, the polypeptides and fragments of the present invention can be produced by direct peptide synthesis using solid-phase techniques (Merrifield, J. Am. Chem. Soc. 85:2149-2154 (1963)). Protein synthesis can be performed manually or automated. Automated synthesis can be achieved using, for example, an Applied Biosystems 431A Peptide Synthesizer (Perkin Elmer). Alternatively, various fragments can be chemically synthesized individually and then combined using chemical methods to produce the full-length molecule.

According to another aspect of the present invention, polynucleotides encoding the polypeptides of the present invention can be delivered in vivo to a subject using gene therapy techniques. Gene therapy generally refers to the introduction of heterologous nucleic acids into specific cells or target cells of a mammal, particularly humans, having a disorder or condition that requires such treatment. The nucleic acid is introduced into the selected target cells, and the heterologous DNA is expressed, producing the encoded therapeutic product.

Various viral vectors suitable for gene therapy, as disclosed herein, include adenovirus, herpes virus, vaccinia, adeno-associated virus (AAV), or preferably RNA viruses such as retroviruses. Preferably, the retroviral vector can be a murine or avian retrovirus derivative or a lentiviral vector. A preferred retroviral vector can be a lentiviral vector. Examples of retroviral vectors into which a single exogenous gene can be inserted include, but are not limited to, Moloney murine leukemia virus (MoMuLV), Harvey murine sarcoma virus (HaMuSV), murine mammary tumor virus (MuMTV), SIV, BIV, HIV, and Rous Sarcoma Virus (RSV). Many additional retroviral vectors can incorporate multiple genes. All these vectors can include selectable marker genes to allow for the identification and production of transduced cells. For example, by inserting a sequence encoding a desired zinc finger-derived DNA-binding polypeptide along with another gene encoding a ligand for a receptor on specific target cells into a viral vector, the vector can be made target-specific.

Retroviral vectors can be made target-specific by inserting a polynucleotide encoding a polypeptide. For example, targeting can be achieved by using an antibody that targets the retroviral vector. Those skilled in the art are well-versed in specific polynucleotide sequences that can be inserted into the retroviral genome to deliver a retroviral vector containing a zinc finger-nucleotide binding protein polynucleotide in a target-specific manner, and can easily identify them without excessive experimentation.

Since recombinant retroviruses are defective, assistance is needed to produce infectious vector particles. This assistance can be provided, for example, by using a helper cell line that includes a plasmid encoding all structural genes of the retrovirus under the control of regulatory sequences within the LTR. These plasmids lack nucleotide sequences that enable the packaging mechanism to recognize RNA transcripts for encapsulation. Examples of helper cell lines with defective packaging signals include, but are not limited to, PSI.2, PA317, and PA12. These cell lines produce empty virions because their genomes are not packaged. If a retroviral vector is introduced into cells where the packaging signal is intact and the structural genes are replaced with other desired genes, the vector can be packaged and produce vector virions. The vector virions produced by this method can then be used to infect tissue cell lines (e.g., NIH3T3 cells), thereby producing a large number of chimeric retroviral virions.

Non-viral delivery techniques for gene therapy, such as DNA-ligand complexes, adenovirus-ligand-DNA complexes, direct injection of DNA, CaPO₄ precipitation, gene gun technology, electroporation, liposome methods, and lipofection, can also be used. Any of these methods are widely available to those skilled in the art and are suitable for use in the present invention. Other suitable methods are also available to those skilled in the art, and it is clearly understood that the present invention can be achieved using any available transfection method. Lipofection can be achieved by encapsulating isolated DNA molecules within liposome particles and contacting the liposome particles with the cell membrane of target cells. Liposomes are self-assembling colloidal molecules composed of lipid bilayers formed by amphipathic molecules such as phosphatidylserine or phosphatidylcholine, encapsulating part of the surrounding medium, resulting in a hydrophilic interior surrounded by the lipid bilayer. Unilamellar or multilamellar liposomes can be constructed, containing desired chemicals, drugs, or isolated DNA molecules as in the present invention.

In one aspect of the present invention, the virus may be characterized as an RNA virus. RNA viruses use reverse transcriptase to convert their RNA genetic information into DNA, which is then inserted into the host's DNA, causing the host to replicate the virus's genetic information.

In the present invention, the RNA virus is not particularly limited in type and may be selected from the group consisting of Amalgaviridae, Birnaviridae, Chrysoviridae, Cystoviridae, Endornaviridae, Hypoviridae, Megabirnaviridae, Partitiviridae, Picobirnaviridae, Reoviridae, Totiviridae, Quadriviridae, Arteriviridae, Coronaviridae, Mesoniviridae, Roniviridae, Dicistroviridae, Iflaviridae, Marnaviridae, Picornaviridae, Secoviridae, Alphaflexiviridae, Betaflexiviridae, Gammaflexiviridae, Tymoviridae, Bornaviridae, Filoviridae, Paramyxoviridae, Rhabdoviridae, Nyamiviridae, Caliciviridae, Flaviviridae, Luteoviridae, Togaviridae, Pneumoviridae, Arenaviridae, Deltavirus, and Orthomyxoviridae viruses.

In a preferred aspect of the present invention, the RNA virus may be selected from the group consisting of influenza virus, influenza A virus subtype H1N1, avian influenza virus, rhinovirus, coronavirus, parainfluenza virus, respiratory syncytial virus, human immunodeficiency virus (HIV), retrovirus, and hepatitis C virus.

In the present invention, the coronavirus may be characterized as being selected from the group consisting of severe acute respiratory syndrome coronavirus 2 (SARS-CoV-2), severe acute respiratory syndrome coronavirus (SARS-CoV), and Middle East respiratory syndrome (MERS) coronavirus.

The pharmaceutical composition for antiviral use according to the present invention can be administered as a vaccine for the prevention of viral infections before infection, or as a therapeutic agent for the treatment of viral infections after infection.

The present invention also provides a vaccine adjuvant comprising one or more selected from the group consisting of:
(i) the peptide,
(ii) a polynucleotide encoding (i),
(iii) a vector comprising (ii), and
(iv) a host cell transformed with (iii).

In the present invention, the vaccine adjuvant can be defined as a substance that presents an antigen to the immune system in a manner that induces an immune response or an increase in the immune response to the antigen when the antigen is administered with the vaccine adjuvant. That is, the vaccine adjuvant is a substance that enhances the immune response to an antigen as an immunostimulant, meaning a substance that enhances immunity by increasing the activity of immune system cells, although it is not an immunogen to the host.

To analyze the antigen-specific induced immune response by the vaccine adjuvant, the immune response can be compared to the immune response induced in the presence of the antigen without the vaccine adjuvant. The induction can be evaluated in the subject or in the subject's cells.

In the present invention, the immune response can be selected from the group consisting of macrophage, dendritic cell, monocyte, B cell, and T cell responses. In particular, the immune response can be a B cell response, meaning that it can specifically produce antibodies against the antigen. The antibody is preferably an IgG antibody, more preferably an IgG2a and/or IgG1 antibody. The immune response can be a T cell response, preferably a Th1 response, a Th2 response, or a balanced Th2/Th1 response. Those skilled in the art recognize that B and/or T cell responses, which vary depending on the disease, may need to be induced to control it. Alternatively, the immune response can be detected by measuring the production of cytokines such as IFNgamma, IL-6, TNFalpha, or IL-10. The production of these cytokines can be evaluated by ELISA, preferably as performed in the examples.

According to one embodiment of the present invention, the peptide consisting of the amino acid sequence of SEQ ID NO:2 or an amino acid sequence having 95% or more sequence homology thereto has been confirmed to have a very excellent effect in activating innate and acquired immunity, and it has been confirmed that the increase in the immune response of the subject can be significantly improved when treated with the antigen.

Therefore, it can be clearly understood by those skilled in the art that the peptide consisting of the amino acid sequence of SEQ ID NO:2 or an amino acid sequence having 95% or more sequence homology thereto, a polynucleotide encoding the same, a vector comprising the polynucleotide, and a host cell transformed with the vector will also exhibit the same physiological activity.

As one embodiment of the present invention, the vaccine adjuvant can induce improved activation of innate and acquired immune responses when administered together with the antigen compared to when the antigen is obtained alone, and more specifically, it can induce improved activation of cellular and humoral immune responses.

As one embodiment of the present invention, the detection of the antigen-specific induced immune response means that the detection occurs at least 1, 2, 3, 4, 5, 6, 7, 8, 9, 10, 11, 12 hours or more after administering the vaccine adjuvant of the present invention, or at least 1 day, or at least 2 days, or at least 3 days, or at least 4 days or more after. The detection can be evaluated in the subject or the subject's cells, preferably as performed in the examples.

As one embodiment of the present invention, the antigen-specific induced immune response preferably means a detectable immune response to the antigen. A detectable increase means that the amount of immune cells, antibodies, and/or cytokines increases by at least 5%, or 10%, 15%, 20%, 25%, 30%, 35%, 40%, 45%, 50%, 55%, 60%, 65%, 70%, 75%, 80%, 85%, 90%, 95%, 100%, 150%, 200% or more after administering the vaccine adjuvant and antigen to the subject, at least 1, 2, 3, 4, 5, 6, 7, 8, 9, 10, 11, 12 hours or more, or at least 1 day, or at least 2 days, or at least 3 days, or at least 4 days or more. The detection can be evaluated in the subject or the subject's cells, preferably as performed in the examples.

The present invention also provides a vaccine composition comprising the vaccine adjuvant and the antigen.

In the present invention, the vaccine refers to a formulation used to confer immunity against a disease caused by an organism, containing an antigen consisting of the whole disease-causing organism (either structured or attenuated) or components thereof, such as proteins, peptides, or polysaccharides. As described above, a peptide consisting of the amino acid sequence of SEQ ID NO:2; or a peptide comprising an amino acid sequence having 95% or more homology with the peptide can be utilized as a vaccine composition by significantly enhancing the immune response of the subject when administered together with the antigen by activating the innate and acquired immune systems.

In the present invention, the antigen included in the vaccine composition is not particularly limited and can be peptides, proteins, glycoproteins, glycolipids, lipids, carbohydrates, nucleic acids, polysaccharides, and viruses, bacterial cells, allergens, tissues, cells containing them. Non-limiting examples include antigens derived from pollen, hepatitis A virus, hepatitis B virus, hepatitis C virus, hepatitis D virus, hepatitis E virus, hepatitis F virus, HIV virus, influenza virus, herpes virus (HSV-1, HSV-2), anthrax bacterium, chlamydia, pneumococcus, Japanese encephalitis virus, measles virus, rubella virus, tetanus bacterium, varicella virus, SARS virus, EB virus, papilloma virus, Helicobacter pylori, rabies virus, West Nile virus, hantavirus, streptococcus, staphylococcus, Bordetella pertussis, Mycobacterium tuberculosis, Plasmodium, poliovirus, various zoonotic infections, cancer antigens, various food allergy antigens, etc.

The antigen included in the vaccine composition of the present invention does not need to be single. Considering the application of the present invention, it may induce an immune response to multiple components such as cancer cells, bacteria, viruses, etc., rather than a single protein or peptide. In this case, it may be a mixture of multiple types of proteins or other substances that can induce an immune response, even if the types cannot be specified. Additionally, it is also an application form of the vaccine composition of the present invention to actively induce an immune response to multiple types of antigens by including multiple types of antigens.

The antigen included in the vaccine composition of the present invention may preferably be a tumor antigen. Tumor antigens are tumor-specific antigens (TSA) or tumor-associated antigens (TAA). Various tumor antigens and their expression patterns are well known in the art and can be selected according to the type of tumor to be treated. Non-limiting examples of tumor antigens include alpha-fetoprotein, carcinoembryonic antigen, cdk4, beta-catenin, CA125, caspase-8, epithelial tumor antigen, HPV antigen, HPV16 antigen, CTL epitope derived from HPV16 E7 antigen, melanoma-associated antigen (MAGE)-1, MAGE-3, tyrosinase, surface Ig idiotype, Her-2/neu, MUC-1, prostate-specific antigen (PSA), sialyl Tn (STn), heat shock protein, gp96, ganglioside molecule GM2, GD2, GD3, carcinoembryonic antigen (CEA), PRAME, WT1, survivin, cyclin D, cyclin E, HER2, MAGE, NY-ESO, EGF, GP100, cathepsin G, human papillomavirus (HPV)-16-E6, HPV-16-E7, HPV-18-E6, HPV-18-E7, Her/2-neu antigen, chimeric Her2 antigen, prostate-specific antigen (PSA), bivalent PSA, ERG, androgen receptor (AR), PAK6, prostate stem cell antigen (PSCA), NY-ESO-1, Stratum Corneum Chymotryptic Enzyme (SCCE) antigen, Wilms tumor antigen 1 (WT-1), HIV-1 gag, human telomerase reverse transcriptase (hTERT), proteinase 3, tyrosinase-related protein 2 (TRP2), high molecular weight melanoma-associated antigen (HMW-MAA), synovial sarcoma, X (SSX)-2, carcinoembryonic antigen (CEA), melanoma-associated antigen E (MAGE-A, MAGE1, MAGE2, MAGE3, MAGE4), interleukin-13 receptor alpha (IL13-R alpha), carbonic anhydrase IX (CAIX), survivin, GP100, angiogenesis antigen, ras protein, p53 protein, p97 melanoma antigen, KLH antigen, carcinoembryonic antigen (CEA), gp100, MART1 antigen, TRP-2, HSP-70, beta-HCG, testisin, 1A01_HLA-A/m; 1A02; 5T4; ACRBP; AFP; AKAP4; alpha-actinin-_4/m; alpha-methylacyl-CoAracemase; ANDR; ART-4; ARTC1/m; AURKB; B2MG; B3GN5; B4GN1; B7H4; BAGE-1; BASI; BCL-2; bcr/abl; beta-catenin/m; BING-4; BIRC7; BRCA1/m; BY55; calreticulin; CAMEL; CASPA; caspase_8; cathepsin_B; cathepsin_L; CD1A; CD1B; CD1C; CD1D; CD1E; CD20; CD22; CD276; CD33; CD3E; CD3Z; CD4; CD44_isoform_1; CD44_isoform_6; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen _XXIII; COX-2; CP1B1; CSAG2; CT-_9/BRD6; CT45A1; CT55; CTAG2 _isoform_LAGE-1A; CTAG2 _isoform_LAGE-1B; CTCFL; Cten; cyclin_B1; cyclin_D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_version_1; FCGR3A_version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8_(GAGE-2D); GASR; GnT-V; GPC3; GPNMB/m; GRM3; HAGE; hepsin; Her2/neu; HLA-A2/m; homeobox _NKX3.1; HOM-TES-85; HPG1; HS71A; HS71B; HST-2; hTERT; iCE; IF2B3; IL-10; IL-13Ra2; IL2-RA; IL2-RB; IL2-RG; IL-5; IMP3; ITA5; ITB1; ITB6; kallikrein-2; kallikrein-4; KI20A; KIAA0205; KIF2C; KK-LC-1; LDLR; LGMN; LIRB2; LY6K; MAGA5; MAGA8; MAGAB; MAGE- _B1; MAGE- E1; MAGE-A1; MAGE-A10; MAGE-A12; MAGE-A2; MAGE-A3; MAGE-A4; MAGE-A6; MAGE-A9; MAGE-B10; MAGE-B16; MAGE-B17; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-E1_(MAGE1); MAGE-E2; MAGE-F1; MAGE-H1; MAGEL2; mammaglobin_A; MART-1/melan-A; MART-2; MC1_R; M-CSF; mesothelin; MITF; MMP1_1; MMP7; MUC-1; MUM-1/m; MUM-2/m; MYO1A; MYO1B; MYO1C; MYO1D; MYO1E; MYO1F; MYO1G; MYO1H; NA17; NA88-A; Neo-PAP; NFYC/m; NGEP; N-myc; NPM; NRCAM; NSE; NUF2; NY-ESO-1; OA1; OGT; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1_-kinase; Pin-1; PLAC1; PMEL; PML; POTE; POTEF; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-1; SART-2; SART-3; SEPR; SERPINB5; SIA7F; SIA8A; SIAT9; SIRT2/m; SOX10; SP17; SPNXA; SPXN3; SSX-1; SSX-2; SSX3; SSX-4; ST1A1; STAG2; STAMP-1; STEAP-1; survivin; survivin-2B; SYCP1; SYT-SSX-1; SYT-SSX-2; TARP; TCRg; TF2AA; TGFbeta1; TGFR2; TGM-4; TIE2; TKTL1; TPI/m; TRGV11; TRGV9; TRPC1; TRP-p8; TSG10; TSPY1; TVC_(TRGV3); TX101; tyrosinase; TYRP1; TYRP2; UPA; VEGFR1; WT1; XAGE1, alpha-actinin-4; ARTC1; BCR-ABL fusion protein (b3a2); B-RAF; CASP-5; CASP-8; beta-catenin; Cdc27; CDK4; CDKN2A; COA-1; dek-can fusion protein; EFTUD2; elongation factor 2; ETV6-AML1 fusion protein; FN1; GPNMB; LDLR-fucosyltransferase AS fusion protein; HLA-A2d; HLA-A11d; hsp70-2; KIAAO205; MART2; ME1; MUM-If; MUM-2; MUM-3; neo-PAP; myosin class I; NFYC; OGT; OS-9; pml-RAR alpha fusion protein; PRDX5; PTPRK; K-ras; N-ras; RBAF600; SIRT2; SNRPD1; SYT-SSX1 or SSX2 fusion protein; triosephosphate isomerase; BAGE-1; GAGE-1,2,8; GAGE-3,4,5,6,7; GnTVf; HERV-K-MEL; KK-LC-1; KM-HN-1; LAGE-1; MAGE-A1; MAGE-A2; MAGE-A3; MAGE-A4; MAGE-A6; MAGE-A9; MAGE-A10; MAGE-A12; MAGE-C2; mucin k; NA-88; NY-ESO-1 / LAGE-2; SAGE; Sp17; SSX-2; SSX-4; TRAG-3; TRP2-INT2g; CEA; gp100 / Pmel17; kallikrein 4; mammaglobin-A; Melan-A/ MART-1; NY-BR-1; OA1; PSA; RAB38 / NY-MEL-1; TRP-1 / gp75; TRP-2; tyrosinase; adipophilin; AIM-2; BING-4; CPSF; cyclin D1; Ep-CAM; EphA3; FGF5; G250 / MN / CAIX; HER-2 / neu; IL13R alpha2; intestinal carboxyl esterase; alpha-foetoprotein; M-CSF; mdm-2; MMP-2; MUC1; p53; PBF; PRAME; PSMA; RAGE-1; RNF43; RU2AS; secernin 1; SOX10; STEAP1; survivin; telomerase; WT1; FLT3-ITD; BCLX(L); DKK1; ENAH(hMena); MCSP; RGS5; gastrin-17; human chorionic gonadotropin, EGFRvIII, HER2, HER2/neu, P501, guanylyl cyclase C, PAP, OVA (ovalbumin), and MART-1.

The vaccine composition of the present invention may preferably be an anticancer vaccine. Additionally, the anticancer vaccine may be a vaccine for cancer prevention or a vaccine for cancer treatment.

According to an aspect of the present invention, a peptide consisting of the amino acid sequence of SEQ ID NO:2 or an amino acid sequence having 95% or more sequence homology thereto; and a vaccine composition comprising a specific tumor antigen can exhibit a preventive effect by inhibiting the growth of cancer by pre-administering it before cancer formation to activate the immune response of the subject. Furthermore, the vaccine composition of the present invention has been confirmed to have efficacy as a therapeutic vaccine by inhibiting the growth of cancer or killing cancer when administered to a subject after cancer formation.

In the present invention, the type of cancer is not particularly limited, and preferably, it can be selected from the group consisting of breast cancer, colon cancer, prostate cancer, cervical cancer, stomach cancer, skin cancer, oral cancer, lung cancer, glioblastoma, oral cancer, pituitary adenoma, glioma, brain tumor, nasopharyngeal cancer, laryngeal cancer, thymoma, mesothelioma, esophageal cancer, rectal cancer, liver cancer, pancreatic cancer, pancreatic neuroendocrine tumor, gallbladder cancer, penile cancer, ureteral cancer, renal cell carcinoma, bladder cancer, non-Hodgkin's lymphoma, myelodysplastic syndrome, multiple myeloma, plasmacytoma, leukemia, pediatric cancer, bronchial cancer, colon cancer, and ovarian cancer.

The vaccine composition of the present invention may further comprise one or more selected from the group consisting of any vaccine adjuvant and immune checkpoint inhibitor.

According to one embodiment of the present invention, it has been confirmed that the peptide of the present invention has a more remarkable effect in activating the immune function of the subject when treated in combination with any additional vaccine adjuvant and/or immune checkpoint inhibitor. In particular, this effect can be especially desirable in that it minimizes side effects that may occur by administering a high dose of the substance and achieves the maximum effect with minimal administration.

The vaccine adjuvant that can be additionally included in the vaccine composition of the present invention is not particularly limited in type, and it should be understood that vaccine adjuvants currently widely used in the art or newly developed vaccine adjuvants in the future are also included. Non-limiting examples of the arbitrary vaccine adjuvant include 1018 ISS, aluminum salts, Amplivax, AS15, BCG, CP-870,893, CpG ODN, CpG7909, CyA, dSLIM, GM-CSF, IC30, IC31, Imiquimod, ImmuFact IMP321, IS patch, Iscomatrix, JuviMune, LipoVac, MF59, monophosphoryl lipid A, Montanide IMS 1312, Montanide ISA 206, Montanide ISA 50V, Montanide ISA-51, OK-432, OM-174, OM-197-MP-EC, Ontak, Peptivator system, PLG microparticles, resiquimod, SRL172, virosomes and other virus-like particles, YF-17D BCG, Aquila's QS21 Stimulon, Ribi Detox Quil, Superfos, Freund's, GM-CSF, cholera toxin, immunological adjuvants, MF59, and cytokines, and most preferably CpG ODN.

In the present invention, the immune checkpoint inhibitor is a substance that blocks the inhibitory checkpoint of the immune system. The immune checkpoint can be stimulatory or inhibitory. Blocking the inhibitory immune checkpoint activates the immune system function and can be used in cancer immunotherapy [see: Pardoll, Nature Reviews. Cancer 12:252-64 (2012)]. Tumor cells, when attached to specific T-cell receptors, deplete activated T cells. Immune checkpoint inhibitors prevent tumor cells from attaching to T cells, keeping T cells in an activated state. In fact, the cooperative action by cells and soluble components fights damage caused by pathogens and cancer. Regulation of immune pathways involves altering the expression or functional activity of at least one component of the pathway to modulate the response by the immune system. The immune checkpoint inhibitors can be selected from the group consisting of PD-1 (programmed cell death-1) antagonists, PD-L1 (programmed cell death-ligand 1) antagonists, PD-L2 (programmed cell death-ligand 2) antagonists, CD27 (cluster of differentiation 27) antagonists, CD28 (cluster of differentiation 28) antagonists, CD70 (cluster of differentiation 70) antagonists, CD80 (cluster of differentiation 80, also known as B7-1) antagonists, CD86 (cluster of differentiation 86, also known as B7-2) antagonists, CD137 (cluster of differentiation 137) antagonists, CD276 (cluster of differentiation 276) antagonists, KIRs (killer-cell immunoglobulin-like receptors) antagonists, LAG3 (lymphocyte-activation gene 3) antagonists, TNFRSF4 (tumor necrosis factor receptor superfamily, member 4, also known as CD134) antagonists, GITR (glucocorticoid-induced TNFR-related protein) antagonists, GITRL (glucocorticoid-induced TNFR-related protein ligand) antagonists, 4-1BBL (4-1BB ligand) antagonists, CTLA-4 (cytolytic T lymphocyte associated antigen-4) antagonists, A2AR (Adenosine A2A receptor) antagonists, VTCN1 (V-set domain-containing T-cell activation inhibitor 1) antagonists, BTLA (B- and T-lymphocyte attenuator) antagonists, IDO (Indoleamine 2,3-dioxygenase) antagonists, TIM-3 (T-cell Immunoglobulin domain and Mucin domain 3) antagonists, VISTA (V-domain Ig suppressor of T cell activation) antagonists, and KLRA (killer cell lectin-like receptor subfamily A) antagonists, preferably PD-1 antagonists, PD-L1 antagonists, or LAG3 antagonists, and most preferably PD-L1 antagonists.

In one embodiment of the present invention, the immune checkpoint inhibitor may be a PD-1 antagonist. The PD-1 is a T-cell co-inhibitory receptor that plays a central role in the ability of tumor cells to evade the host's immune system. Blocking the interaction between PD-1 and its ligands, PD-L1, PD-1 antagonists enhances immune function and mediates antitumor activity. Examples of the PD-1 antagonist include antibodies that specifically bind to PD-1. Specific anti-PD-1 antibodies include nivolumab, pembrolizumab, STI-1014, and pidilizumab, but are not limited thereto.

In another embodiment of the present invention, the immune checkpoint inhibitor may be a PD-L1 antagonist. Examples of the PD-L1 antagonist include antibodies that specifically bind to PD-L1. Specific anti-PD-L1 antibodies include avelumab, atezolizumab, durvalumab, and BMS-936559, but are not limited thereto.

In yet another embodiment, the immune checkpoint inhibitor may be a LAG3 antagonist. LAG3, lymphocyte activation gene 3, is a negative co-stimulatory receptor that regulates T cell homeostasis, proliferation, and activation. It has also been reported that LAG3 is involved in the suppressive function of regulatory T cells (Treg). Most of the LAG3 molecules are retained in cells near the microtubule-organizing center and are induced only after antigen-specific T cell activation [see: U.S. 2014/0286935]. Examples of the LAG3 antagonist include antibodies that specifically bind to LAG3. Specific anti-LAG3 antibodies include GSK2831781, but are not limited thereto.

In the present invention, the antibody means an intact monoclonal antibody, polyclonal antibody, multispecific antibody formed from at least two intact antibodies, and antibody fragments, as long as they exhibit the intended biological activity. In another embodiment, the antibody means a soluble receptor that does not retain the Fc portion of the antibody. In one embodiment, the antibody may be a humanized monoclonal antibody and its fragment produced by recombinant genetic engineering.

Another class of immune checkpoint inhibitors includes polypeptides that bind to and block the PD-1 receptor on T cells without inducing inhibitory signal transduction. Such peptides include B7-DC polypeptides, B7-H1 polypeptides, B7-1 polypeptides, and B7-2 polypeptides and their soluble fragments, as disclosed in U.S. Patent No. 8,114,845.

Another class of immune checkpoint inhibitors includes compounds with peptide moieties that inhibit PD-1 signaling. Examples of such compounds are generally disclosed in the known literature.

Another class of immune checkpoint inhibitors includes inhibitors of specific metabolic enzymes such as indoleamine 2,3-dioxygenase (IDO), which are expressed by infiltrating bone marrow cells and tumor cells. The IDO enzyme suppresses immune responses by depleting amino acids necessary for anabolism in T cells or through the synthesis of specific natural ligands for cytoplasmic receptors that can alter lymphocyte function.

The compositions of the present invention (e.g., polypeptides, polynucleotides, etc.) can generally be formulated in a pharmaceutically acceptable or physiologically acceptable solution for administration to cells, tissues, or animals, either alone or in combination with one or more other therapeutic modalities. If desired (as needed), the compositions of the present invention can be administered in combination with other drugs (e.g., other proteins, polypeptides, or various pharmaceutically active drugs). There are no limitations on other components that can be included in the compositions of the present invention, as long as the additional drugs do not negatively affect the properties of the peptides of the present invention.

The route of administration can be oral or parenteral. Parenteral administration methods include, but are not limited to, intranasal, intravenous, intramuscular, intra-arterial, intramedullary, intradural, intracardiac, transdermal, subcutaneous, intraperitoneal, intranasal, enteral, topical, sublingual, or rectal administration.

When the pharmaceutical composition of the present invention is administered orally, the pharmaceutical composition of the present invention can be formulated into forms such as powders, granules, tablets, pills, sugar-coated tablets, capsules, liquids, gels, syrups, suspensions, and wafers according to methods known in the art, along with suitable oral administration carriers. Examples of suitable carriers include sugars such as lactose, dextrose, sucrose, sorbitol, mannitol, xylitol, erythritol, and maltitol; starches such as corn starch, wheat starch, rice starch, and potato starch; celluloses such as cellulose, methyl cellulose, sodium carboxymethyl cellulose, and hydroxypropyl methyl cellulose; and fillers such as gelatin and polyvinylpyrrolidone. Additionally, cross-linked polyvinylpyrrolidone, agar, alginic acid, or sodium alginate may be added as disintegrants. Furthermore, the pharmaceutical composition may additionally comprise anti-caking agents, lubricants, wetting agents, flavoring agents, emulsifiers, and preservatives.

Also, when administered parenterally, the pharmaceutical composition of the present invention can be formulated into forms such as injectables, transdermal administration agents, and nasal inhalants according to methods known in the art, along with suitable parenteral administration carriers. In the case of injectables, they must be sterile and protected from contamination by microorganisms such as bacteria and fungi. Examples of suitable carriers for injectables include, but are not limited to, solvents or dispersion media comprising water, ethanol, polyols (e.g., glycerol, propylene glycol, and liquid polyethylene glycol), their mixtures, and/or vegetable oils. More preferably, suitable carriers include Hank's solution, Ringer's solution, phosphate-buffered saline (PBS) containing triethanolamine, sterile water for injection, isotonic solutions such as 10% ethanol, 40% propylene glycol, and 5% dextrose. To protect the injectables from microbial contamination, various antibacterial and antifungal agents such as parabens, chlorobutanol, phenol, sorbic acid, and thimerosal may be additionally included. Additionally, the injectables may further comprise isotonic agents such as sugars or sodium chloride in most cases.

For transdermal administration, forms such as ointments, creams, lotions, gels, external solutions, pastes, liniments, and aerosols are included. "Transdermal administration" means that the pharmaceutical composition is topically applied to the skin, allowing an effective amount of the active ingredient contained in the pharmaceutical composition to be delivered into the skin. For example, the pharmaceutical composition of the present invention can be prepared in an injectable form and administered by lightly pricking the skin with a fine 30-gauge needle or by directly applying it to the skin. These formulations are described in literature generally known in pharmaceutical chemistry.

For inhalation administration, the compounds used in the present invention can be conveniently delivered in the form of an aerosol spray from a pressurized pack or nebulizer using a suitable propellant, such as dichlorofluoromethane, trichlorofluoromethane, dichlorotetrafluoroethane, carbon dioxide, or other suitable gases. In the case of pressurized aerosols, the dosage unit can be determined by providing a valve that delivers a metered amount. For example, gelatin capsules and cartridges used in inhalers or insufflators can be formulated to contain a powder mixture of the compound and a suitable powder base such as lactose or starch.

Other pharmaceutically acceptable carriers can be referenced from known literature.

Additionally, the pharmaceutical composition of the present invention may further comprise one or more buffers (e.g., saline or PBS), carbohydrates (e.g., glucose, mannose, sucrose, or dextran), antioxidants, bacteriostats, chelating agents (e.g., EDTA or glutathione), adjuvants (e.g., aluminum hydroxide), suspending agents, thickeners, and/or preservatives.

Furthermore, the pharmaceutical composition of the present invention can be formulated using methods known in the art to provide rapid, sustained, or delayed release of the active ingredient after administration to a mammal.

The pharmaceutical composition of the present invention can also be administered in combination with known substances having antiviral effects.

In the composition of the present invention, the combination of pharmaceutically acceptable excipients and carriers is well known to those skilled in the art. Additionally, appropriate dosing and therapeutic regimens for the use of specific compositions described herein can be employed using well-known methods, including oral, parenteral, intravenous, intranasal, intracerebral, and intramuscular administration and formulations for such administration.

In certain applications, the pharmaceutical composition disclosed herein can be delivered to a subject by oral administration. In such cases, the composition can be formulated with inert diluents or absorbable edible carriers, or the composition can be enclosed in hard-shell or soft-shell gelatin capsules, compressed into tablets, or directly incorporated into food.

In specific situations, the pharmaceutical composition disclosed herein may preferably be delivered parenterally, intravenously, intramuscularly, or intraperitoneally, and such routes of administration can be referenced from known literature. Solutions of the active compound (as a free base or pharmaceutically acceptable salt) can be prepared by appropriately mixing with a surfactant such as hydroxypropylcellulose in water. Dispersions can also be prepared in glycerol, liquid polyethylene glycol, or their mixtures, or in oils. To prevent microbial growth under common storage and use conditions, the preparations may include preservatives.

Pharmaceutical forms suitable for injectable use include sterile aqueous solutions or dispersions and sterile powders for the extemporaneous preparation of sterile injectable solutions or dispersions. In all cases, the pharmaceutical form must be sterile and fluid enough to be easily injectable. It must be stable under manufacturing and storage conditions and preserved against contamination by microorganisms such as bacteria and fungi. The carrier can be a solvent or dispersion medium comprising, for example, water, ethanol, polyols (e.g., glycerol, propylene glycol, and liquid polyethylene glycol), their suitable mixtures, and/or vegetable oils. Suitable fluidity can be maintained by using a coating agent such as lecithin, maintaining the required particle size in the case of dispersions, and using surfactants. Prevention of microbial action can be achieved by various antibacterial and antifungal agents (e.g., parabens, chlorobutanol, phenol, sorbic acid, thimerosal). In many cases, it is preferable to include isotonic agents (e.g., sugars or sodium chloride). Prolonged absorption of the injectable compositions can be achieved by using absorption-delaying agents (e.g., aluminum monostearate and gelatin).

For parenteral administration of an aqueous solution, for example, the solution should be appropriately buffered as needed, and the liquid diluent should first be isotonic with sufficient saline or glucose. These specific aqueous solutions are particularly suitable for intravenous, intramuscular, subcutaneous, and intraperitoneal administration. The sterile aqueous media that can be used in this context are well known to those skilled in the art. For example, a single dose can be dissolved in 1 ml of isotonic NaCl solution and added to 1000 ml of hypodermoclysis fluid or injected at the proposed infusion site (for example, see Remington's Pharmaceutical Sciences, 15th Edition, pp. 1035-1038 and 1570-1580). The dosage may vary depending on the symptoms of the subject being treated with the pharmaceutical composition. Those skilled in the art can determine the appropriate dosage for individual subjects using conventional knowledge in the field. Additionally, for administration to humans, the preparations must meet the sterility, pyrogenicity, and general safety and purity standards required by the FDA Office of Biologics.

Sterile injection solutions can be prepared by including the active compound in the required amount in an appropriate solvent, along with various other components listed above, and then sterilizing by filtration as needed. Generally, dispersions can be prepared by including the sterile active ingredients in a sterile vehicle containing the base dispersion medium and other necessary components listed above. **In** the case of sterile powders for the preparation of sterile injection solutions, preferred manufacturing methods may include vacuum-drying and freeze-drying techniques, which produce a powder of the active ingredient along with any additional desired components from the pre-sterilized solution.

The compositions disclosed herein can be formulated in neutral or salt forms. Pharmaceutically acceptable salts may include acid addition salts (formed with free amino groups of proteins), which can be formed with inorganic acids (e.g., hydrochloric or phosphoric acid) or organic acids (e.g., acetic, oxalic, tartaric, mandelic acid). Salts formed with free carboxyl groups can also be derived from inorganic bases (e.g., sodium hydroxide, potassium hydroxide, ammonium hydroxide, calcium hydroxide, or ferric hydroxide) and organic bases (e.g., isopropylamine, trimethylamine, histidine, procaine). Depending on the formulation, the solution is administered in a manner appropriate for the dosage form and in a therapeutically effective amount. The formulation can be easily administered in various dosage forms, such as injection solutions or drug-release capsules.

The term "carrier" as used herein includes any solvent, dispersion medium, vehicle, coating, diluent, antimicrobial and antifungal agent, isotonic agent, and absorption delaying agent, buffer, carrier solution, suspension, colloid, etc. The use of these media and agents for pharmaceutical active substances is well known in the art. They are used in therapeutic compositions unless they are incompatible with the active ingredient. Auxiliary active ingredients may also be included in the compositions of the present invention.

The term "pharmaceutically acceptable" refers to molecular entities and compositions that do not cause allergic or similar adverse reactions when administered to humans. Methods for preparing aqueous compositions containing proteins as active ingredients are well known in the art. Typically, such compositions are prepared as injectable liquid solutions or suspensions; solid forms suitable for dissolution or suspension in liquid prior to injection can also be prepared. Additionally, the preparations can be emulsified.

In certain embodiments, the pharmaceutical composition can be delivered by nasal spray, inhalation, and/or other aerosol delivery vehicles. Methods for delivering gene, polynucleotide, and peptide compositions directly to the lungs via nasal aerosol spray can be referenced in known literature. Similarly, drug delivery using nasal microparticle resins and lysophosphatidyl-glycerol compounds is also well known in the pharmaceutical field. Likewise, transmucosal drug delivery in the form of a polytetrafluoroethylene support matrix can be referenced in known literature.

In certain embodiments, the delivery can be performed using liposomes, nanocapsules, microparticles, microspheres, lipid particles, vesicles, etc., to introduce the compositions of the present invention into appropriate cells. In particular, the compositions of the present invention can be formulated for delivery encapsulated in lipid particles, liposomes, vesicles, nanospheres, or nanoparticles. The formulation and use of these delivery vehicles can be performed using known prior art techniques.

Additionally, the pharmaceutical compositions of the present invention can be formulated using methods known in the art to provide rapid, sustained, or delayed release of the active ingredient after administration to a mammal. The pharmaceutical compositions formulated in this manner can be administered in an effective amount through various routes, including oral, transdermal, subcutaneous, intravenous, or intramuscular, as described above.

The term "effective amount" refers to the amount of substance that enables the tracking of diagnostic or therapeutic effects when administered to a patient, and the term "subject" can include animals, preferably mammals, particularly humans, and may also include cells, tissues, organs derived from animals. The subject may be a patient in need of the aforementioned effects.

The pharmaceutical composition comprising the polypeptide of the present invention may vary in the content of the active ingredient depending on the severity of the disease, but typically, for adults, it can be administered several times a day in an effective dose of 0.1 µg to 10,000 mg, preferably 1 mg to 5,000 mg per dose. However, the dosage of the pharmaceutical composition according to the present invention can be appropriately selected depending on the route of administration, the target, the target disease and its severity, age, gender, body weight, individual differences, and disease state, and such techniques are known to those skilled in the art.

In another aspect, the present invention provides a method of using the compositions of the present invention (e.g., polynucleotides, polypeptides, etc.) in cells, tissues, or subjects to achieve the desired cellular effect and/or therapeutic effect. The cells or tissues that can be regulated by the present invention are preferably mammalian cells or tissues, more preferably human cells or tissues. These cells or tissues may be in a healthy state or a diseased state.

The present invention also provides a pharmaceutical composition for preventing or treating cancer, comprising one or more selected from the group consisting of the following (i) to (iv):
The present invention also provides a pharmaceutical composition for preventing or treating cancer, consisting of one or more selected from the group consisting of the following (i) to (iv):
The present invention also provides a pharmaceutical composition for preventing or treating cancer, consisting essentially of one or more selected from the group consisting of the following (i) to (iv):
(i) the peptide,
(ii) a polynucleotide encoding (i),
(iii) a vector comprising (ii), and
(iv) a host cell transformed with (iii).

According to one aspect of the present invention, the peptide consisting of the amino acid sequence of SEQ ID NO:2 or an amino acid sequence having 95% or more sequence homology thereto can exhibit effects of inhibiting and killing tumor growth by itself, thereby showing cancer prevention or treatment effects.

The 'treatment' in the present invention comprehensively refers to improving symptoms caused by cancer, viral diseases, or the aforementioned diseases, which may include curing the disease, substantially preventing it, or improving the condition. It includes alleviating, curing, or preventing one or most of the symptoms derived from the disease, but is not limited thereto.

### EFFECT OF THE INVENTION

The peptide disclosed in the present invention is a CRS fragment first disclosed in this specification, exhibiting anticancer activity and immune function enhancement activity.

Additionally, the peptide, a polynucleotide encoding the peptide, a vector comprising the polynucleotide, a host cell transformed with the vector, or the full-length CRS protein exhibit excellent anticancer activity and immune function enhancement activity, making them highly useful for the development of vaccine adjuvants, vaccine compositions, and compositions for cancer treatment.

Furthermore, the antiviral composition provided by the present invention not only has the effect of preventing and treating viral infections but also exhibits excellent effects in assisting or enhancing immune responses when administered with viral antigens, making it highly useful for the prevention or treatment of viral infections or the manufacture of viral vaccines.

### BRIEF DESCRIPTION OF THE DRAWINGS

Figure 1A to 1B show the results of confirming the immunoactive function of various fragment proteins derived from CRS (CRS=CARS1).
Figure 1A is a schematic diagram listing the sequences of various fragment proteins derived from CRS.
Figure 1B shows the results of treating Thp-1 cells with PMA (50ng/ml) for 48 hours to induce differentiation, then treating with various fragment proteins derived from CRS (100 nM), and confirming the amount of TNF-α in the medium after 4 hours using ELISA.
Figure 2 is a schematic diagram showing the mutation positions of fragment proteins derived from CRS, specifically a schematic diagram for substituting the cysteine at the 182nd amino acid of the helix3-4 protein derived from CRS with serine.
Figure 3 shows the results of confirming the expression and purification process of CRS (140-200, C182S) protein, where the pET28a CRS (140-200, C182S) gene was transformed into BL21 (DE3) codon plus RIPL cells, and samples obtained from each affinity chromatography purification process were subjected to SDS-PAGE, followed by Coomassie staining of the gel for 1 hour.
Figure 4 shows the results of confirming the polymorphism of CRS (140-200) and CRS (140-200, C182S) proteins, specifically the experimental results confirming whether a monomer, rather than a multimer due to disulfide bonds, appears when the cysteine at the 182nd amino acid of the purified protein through affinity chromatography is substituted with serine using gel filtration chromatography.
Figure 5 shows the results of confirming the charge difference between CRS (140-200) and CRS (140-200, C182S) proteins, specifically the results of confirming the presence of multimers and monomers through ion exchange chromatography by separating the charge difference of the proteins isolated through gel filtration chromatography.
Figure 6A to 6B show the results of confirming the thermal stability and immunoactivity of fragment proteins derived from CRS.

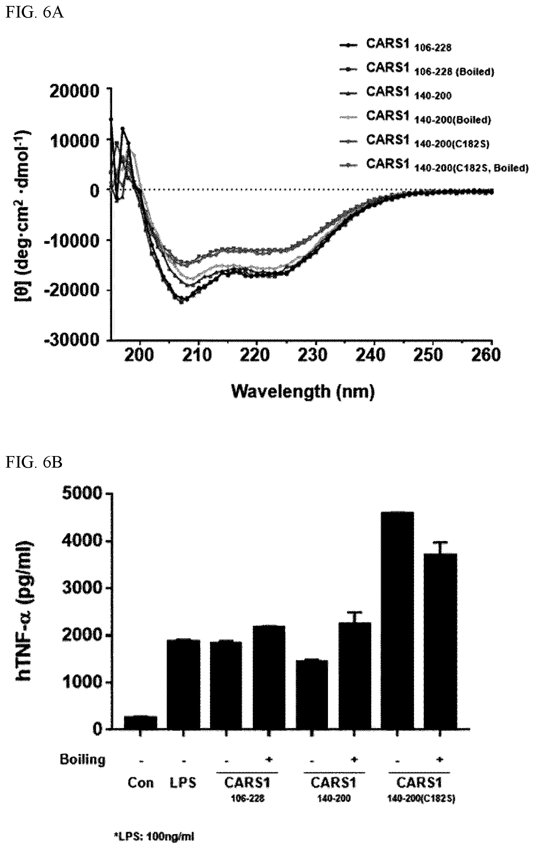

Figure 6A shows the results of analyzing the secondary structure changes of proteins induced by high-temperature denaturation at 100°C for 60 minutes using circular dichroism analysis.
Figure 6B shows the results of treating Thp-1 cells with PMA (50ng/ml) for 48 hours to induce differentiation, then treating with various fragment proteins derived from CRS (100 nM) induced by high-temperature denaturation at 100°C for 60 minutes, and confirming the amount of TNF-α in the medium after 4 hours using ELISA.

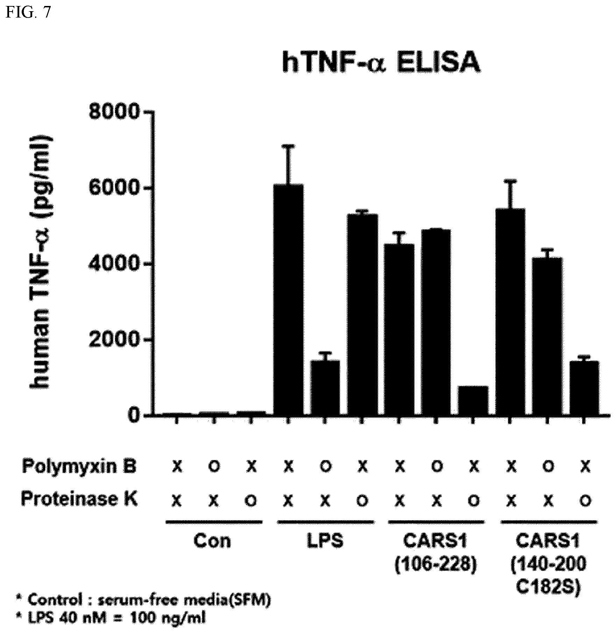

Figure 7 shows the results of confirming the immunoactivity of CRS (140-200, C182S) protein, specifically the results of treating Thp-1 cells with PMA (50ng/ml) for 48 hours to induce differentiation, then treating with polymyxin B (10ug/ml), proteinase K (20ug/ml), and fragment proteins derived from CRS, and confirming the amount of TNF-α in the medium after 4 hours using ELISA.

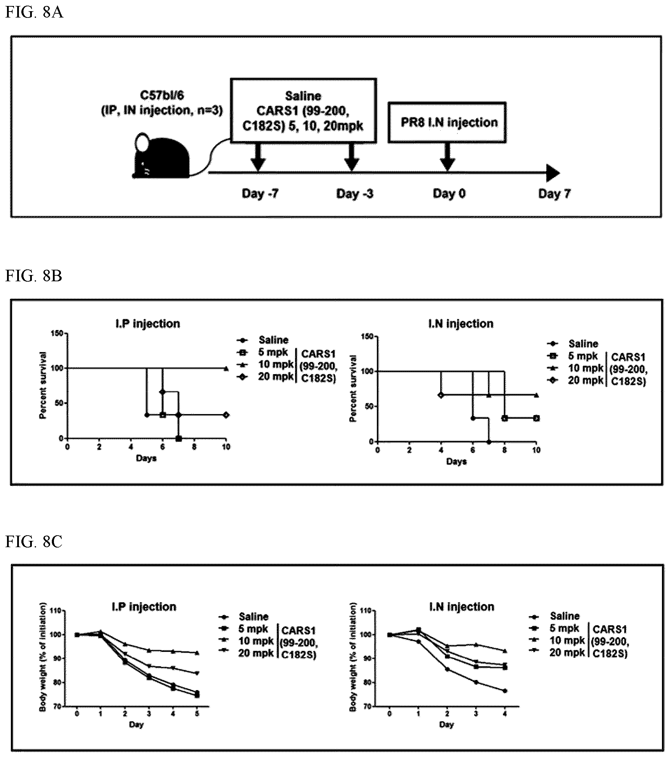

Figure 8A to 8C show the results of confirming the antiviral effect of CARS1 (99-200, C182S) protein according to administration route and concentration (Figure 8A is a schematic diagram briefly showing the experimental schedule. Figures 8B to 8C show the results of administering saline, CARS1 (99-200, C182S) 5mpk, 10mpk, and 20mpk to C57bl/6 mice via intraperitoneal (IP) or intranasal (IN) routes on day-7 and day-0, followed by intranasal administration of PR-8 influenza virus on day-0, and observing survival rate (b) and weight change (c) over time).

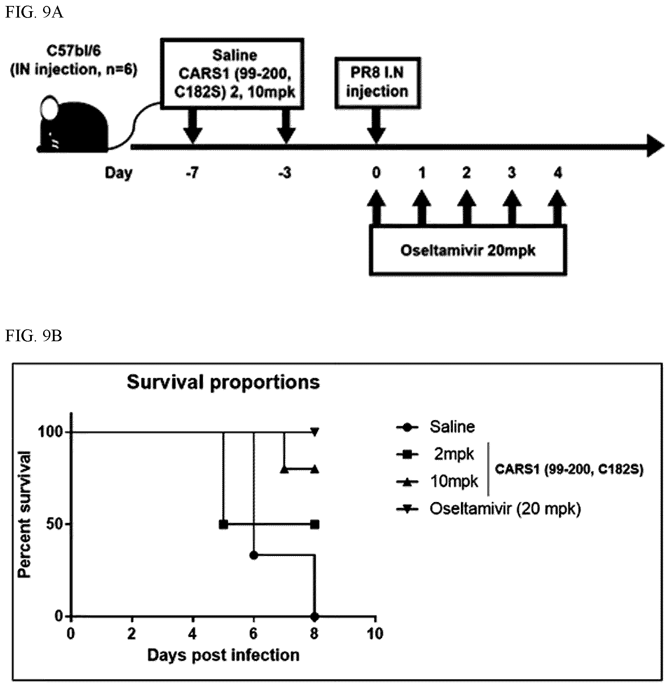

Figure 9A to 9B show the results of confirming the antiviral effect of CARS1 (99-200, C182S) protein compared to the control group (Figure 9A is a schematic diagram briefly showing the experimental schedule. Figure 9B shows the results of administering saline, CARS1 (99-200, C182S) 2mpk or 10mpk, and oseltamivir 20mpk to C57bl/6 mice via intranasal (IN) route, followed by intranasal administration of PR-8 influenza virus, and observing the survival rate).

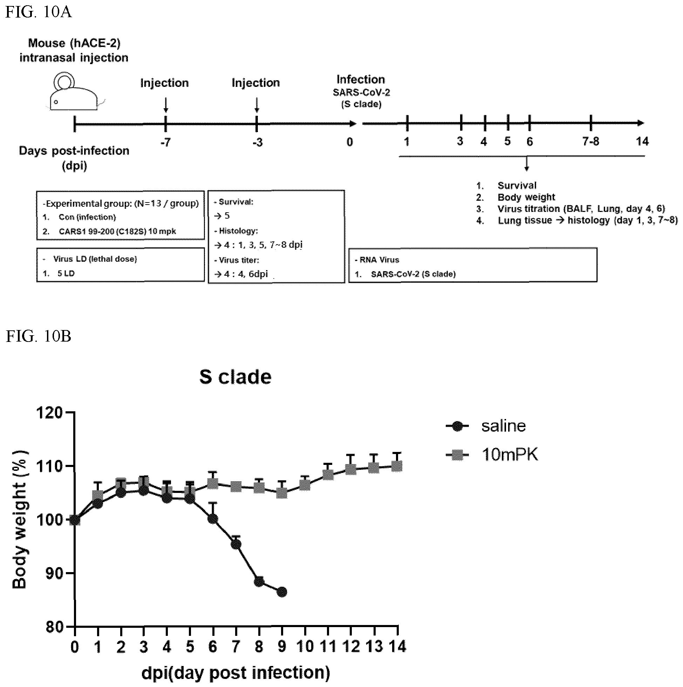

Figure 10A to 10C show the results of confirming the antiviral effect of CARS1 (140-200, C182S) protein (Figure 10A is a schematic diagram briefly showing the experimental schedule. Figures 10B to 10C show the results of administering CARS1 (106-228) and CARS1 (140-200, C182S) 10 mpk to C57bl/6 mice via intraperitoneal route on day -7 and -3, followed by intranasal administration of PR-8 influenza virus on day-0, and observing weight change and survival rate).

### DETAILED DESCRIPTION OF THE INVENTION

Hereinafter, the present invention will be described in detail with reference to the following examples. However, the following examples are merely illustrative of the present invention, and the present invention is not limited thereto.

### Experimental Methods

### 1. Cloning

Various fragment protein genes derived from CRS (=CARS1) were cloned using the pET28a vector containing N, C-terminal 6x his tags. Using CRS DNA as a template, complementary primers were designed at the ends of each sequence, and PCR was performed to obtain PCR products. Next, to insert the PCR products into the target vector, the restriction enzyme NdeI was used for the N-terminal direction, and XhoI was used for the C-terminal direction to restrict the gene, and T4 ligase was used for ligation. The recombinant gene was transformed into DH5α cells to obtain the DNA of the recombinant gene. The transformed cells were cultured on LB agar medium at 37°C for 16 hours, and the formed colonies were inoculated into LB liquid medium and cultured at 37°C for 16 hours. Then, the supernatant was removed by centrifugation at 3000g for 20 minutes, and the precipitate was obtained. The precipitate was lysed using a mini prep kit to obtain DNA, and sequencing was performed through Bioneer to confirm that it matched the designed sequence.

### 2. Affinity Chromatography Purification

The DNA of various proteins derived from CRS was used to transform BL21-codon plus cells, and the colonies were inoculated into the medium and grown. The cells were grown in LB until OD 600 reached 0.5, and protein expression was induced using 0.5 mM IPTG at 4°C for 16 hours. The cell pellet was obtained by centrifugation, and the cells were lysed by sonication in 50 mM Tris buffer pH 7.5 containing 300 mM NaCl. Then, the supernatant was obtained by centrifugation at 20,000g for 30 minutes. This was poured onto a column containing Ni-NTA resin. The washing step was performed with 50 mM Tris, pH 7.5 containing 300 mM NaCl, 5% glycerol, and 15 mM imidazole. The protein was eluted from the column with 10 ml of elution buffer (50 mM Tris pH 7.5, 300 mM NaCl, 5% glycerol, 300 mM imidazole), and endotoxin was removed using TX-114 (REF: Removal of endotoxin from protein solutions by phase separation using Triton X-114). The appropriate protein with less than 0.04 EU/mg from the LAL assay was used for the entire experiment.

### 3. Gel Filtration Chromatography Purification

After attaching a Superdex 75 10/300 GL column to the AKTA pure device, the column was washed with 70% ethanol and 1N NaOH three times the column volume. For column stabilization, 50 mM Tris buffer pH 7.5 containing 150 mM NaCl and 0.1 mM EDTA was passed through the column twice the column volume. 500 µl of protein obtained through affinity chromatography was loaded into a 1 ml syringe and injected into the AKTA injection valve. The protein was eluted at different retention times according to molecular weight. Then, the protein eluted at the same retention time was subjected to SDS-PAGE, and the presence of the target protein was confirmed by coomassie blue staining.

### 4. Ion Exchange Chromatography Purification

After attaching a Hitrap Q column to the AKTA pure device, the column was washed with 70% ethanol and 1N NaOH three times the column volume. For column stabilization, 20 mM Tris buffer pH 7.5 was passed through the column five times the column volume. 500 µl of protein obtained through gel filtration chromatography was loaded into a 1 ml syringe and injected into the AKTA injection valve. Then, the flow rate was set to 5 ml/min, and 20 mM Tris buffer pH 7.5 containing 1M NaCl was passed through to obtain the elution sample in tubes over time.

### 5. ELISA

THP1-PMA cells were tested to confirm cytokine secretion induced by CRS. Each cell type was treated at 5x10⁵ cells/ml in a 24-well plate overnight, and each well was changed to serum-free medium for 2 hours before drug treatment. For RAW264.7 and THP1-PMA, 100 nM of protein was treated for 4 hours. For BMM and BMDC cells, 100 nM of protein was treated for 24 hours. The supernatant was centrifuged at 500g for 10 minutes, and ELISA was performed using IL-6, TNF-α, IL-12 p70, and IL-10 ELISA Set (BD).

### 6. Circular Dichroism Analysis

CD measurement of proteins dissolved in 50 mM Tris buffer containing 300 mM NaCl was performed with a chirascan V100 at a wavelength range of 190-260 nm and a bandwidth condition of 1 nm. A 1 mm pathlength quartz sample cell was used, and the protein to be measured had differences in heat-induced denaturation. Spectrum analysis was performed to analyze changes in the secondary structure of the protein.

### 7. Antiviral Analysis

### (1) Affinity Chromatography Purification (Bacterial Cells)

The DNA of various proteins derived from CARS1 was used to transform BL21-codon plus cells, and the colonies were inoculated into the medium and grown. The cells were grown in LB until OD 600 reached 0.5, and protein expression was induced using 0.5 mM IPTG at 4°C for 16 hours. The cell pellet was obtained by centrifugation, and the cells were lysed by sonication in 50 mM Tris buffer pH 7.5 containing 300 mM NaCl. Then, the supernatant was obtained by centrifugation at 20,000g for 30 minutes. This was poured onto a column containing Ni-NTA resin. The washing step was performed with 50 mM Tris, pH 7.5 containing 300 mM NaCl, 5% glycerol, and 15 mM imidazole. The protein was eluted from the column with 10 ml of elution buffer (50 mM Tris pH 7.5, 300 mM NaCl, 5% glycerol, 300 mM imidazole), and endotoxin was removed using TX-114 (REF: Removal of endotoxin from protein solutions by phase separation using Triton X-114). The appropriate protein with less than 0.04 EU/mg from the LAL assay was used for the entire experiment.

### (2) Trained Immune Mouse Model

### (2-1) Influenza Virus

C57BL/6 mice were purchased from DooYeol Biotech. 6-8 week-old female C57BL/6 mice were subcutaneously injected on the dorsal side with 5, 10, and 20 mg/kg of CARS 1 (99-200, C182S) protein on day-7 and day-3. On day-0, PR8 influenza virus was intranasally administered at LD₅₀ (50% lethal dose). Body weight was measured daily from the day of virus administration, and survival graphs were recorded. In the comparative experiment using Oseltamivir, Oseltamivir was administered orally once daily for 5 days after virus infection.

### (2-2) Coronavirus

hACE-2 introduced C57BL/6 mice were purchased from DooYeol Biotech. 6-8 week-old female hACE-2 introduced C57BL/6 mice were intranasally injected with 10 mg/kg of CARS 1 (99-200, C182S) protein on day-7 and day-3. On day-0, a highly lethal SARS-CoV-2 (S clade) virus was intranasally administered at 5 lethal doses. Body weight was measured daily from the day of virus administration, survival graphs were recorded, and virus titer was analyzed.

### (3) Western Blot

Hek 293T cells were dispensed into a 6-well plate at 3-5 x 10⁵ cells/well and cultured for 24 hours. Then, 1 µg of S-PP-GSAS-Foldon DNA and 2 µl of Terbofect were mixed well and reacted at room temperature for 15-20 minutes. The mixture was then slowly added dropwise to the cells. After 4 hours, the medium was removed, fresh medium was added, and the cells were cultured for 24 hours. 1 ml of the culture medium was taken and centrifuged at 4°C, 500g for 10 minutes. The supernatant was taken and centrifuged at 4°C, 10000g for 30 minutes. 880 µl of the supernatant was separated and carefully mixed with 120 µl of TCA (Trichloroacetic acid solution), and reacted at 4°C for one day. Then, it was centrifuged at 4°C, 18000g for 15 minutes, and the supernatant was completely removed when a precipitate formed, and dried at room temperature for 1 hour. Then, 50 µl of 0.1 M Hepes buffer at pH 8.0 was added to dissolve the precipitate, followed by the addition of 12.5 µl of 5X sample buffer, and boiled at 100°C for 10 minutes. The sample was then subjected to electrophoresis on an 8% polyacrylamide gel and transferred to an Immobilon P PVDF membrane. Anti-Myc antibody and tubulin antibody were then applied, and the target protein was detected using Abclon ECL solution.

### (4) Affinity Chromatography Purification (Animal Cells)

Expi CHO-S cells were resuspended using ExpiCHO Expression medium and cultured in a shaking incubator until they reached 4×10⁶-6×10⁶ cells/ml. One day before transfection, the cells were subcultured to 3×10⁶-4×10⁶ cells/ml. The next day, the cells were diluted to 25 ml with fresh medium to reach 6×10⁶ cells/ml. For transfection, ExpiFectamine CHO Reagent was shaken 4-5 times, and 80 µl was taken and mixed with 920 µl of OptiPRO SFM solution, then pipetted slowly 2-3 times. 20 µg of DNA for transfection was mixed with 1 ml of OptiPRO SFM solution and shaken slowly. Before 5 minutes passed, the solution mixed with ExpiFectamine CHO reagent was slowly added to the DNA solution and shaken slowly. After reacting at room temperature for about 3 minutes, the solution mixed with ExpiFectamine CHO reagent and DNA was slowly added to the flask containing the cells. The flask containing the cells was then incubated at 37°C, 8% CO₂. Between 18-22 hours after incubation, 150 µl of ExpiFectamine CHO Enhancer and 6 ml of ExpiCHO Feed were slowly added, and the cells were cultured under the same conditions. On the 10th day after transfection, all the cell culture medium was taken and placed in a 50 ml centrifuge tube, and centrifuged at 4°C, 5000g for 30 minutes. The supernatant was then taken and passed through a 0.22 µm filter (Acrodisc syringe filter). For affinity chromatography, 3.5 ml of Ni-NTA resin was added to a glass column, and 50 mM Tris binding buffer at pH 8.0 containing 300 mM NaCl and 5% glycerol was passed through the resin at 10 times the resin volume. The previously separated supernatant was then loaded onto the glass column. 100 ml of A wash buffer containing 15 mM imidazole in binding buffer was passed through the glass column, followed by 5 ml of B wash buffer containing 30 mM imidazole in binding buffer. Subsequently, 10 ml of elution buffer containing 300 mM imidazole in binding buffer was loaded onto the column, and the eluate was collected in a 10 ml conical tube. The eluate was then placed in a dialysis cassette and dialyzed against 1.5 L of storage buffer containing 300 mM NaCl and 15% glycerol in 50 mM Tris at pH 8.0 at 4°C for 4 hours. The storage buffer was then replaced with fresh storage buffer (1.5 L), and the dialysis was continued for 16 hours.

### 8. Preparation of Experimental Substances

The CRS fragment peptides used in the present invention and their sequence information are as follows, and these peptides were prepared according to conventional methods:

SEQ ID NO: 2: CRS (140-200)

SEQ ID NO: 3: CRS (140-200, C182S)

SEQ ID NO: 4: CRS (106-228)

SEQ ID NO: 5: CRS (101-200)

SEQ ID NO: 6: CRS (119-200)

SEQ ID NO: 7: CRS (99-200)

SEQ ID NO: 8: CRS (99-200, C182S)

The numbers in parentheses for each sequence indicate the amino acid numbers in the full-length CRS protein of SEQ ID NO: 1. Hereinafter, in the experimental results, CARS1 is interpreted as the same as the above CRS.

### Experimental Results

### 1. Identification of Immunoactive Sites of CRS-Derived Fragment Proteins

To identify the immunoactive sites of the fragment protein containing amino acids 106 to 228 of CRS found in previous studies, several fragment protein genes including each helix were constructed by analyzing the secondary structure of the protein. After culturing and purifying the proteins, they were treated with Thp-1 cells differentiated using PMA.

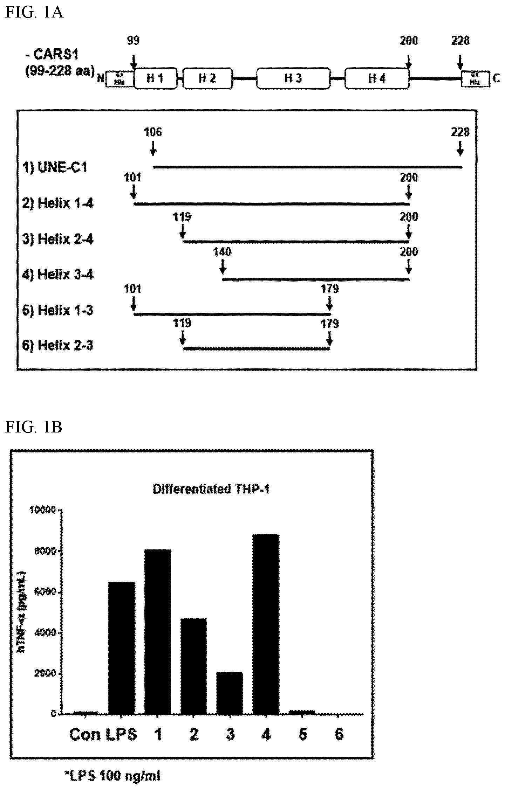

As a result, it was confirmed that proteins not including helix 4 did not induce immune activity compared to LPS. It was confirmed that proteins with sequences 1, 2, 3, and 4, including helices 3 and 4, induced immune activity (Fig. 1A).

### 2. Confirmation of Production Stability of CRS-Derived Fragment Protein CRS (140-200, C182S)

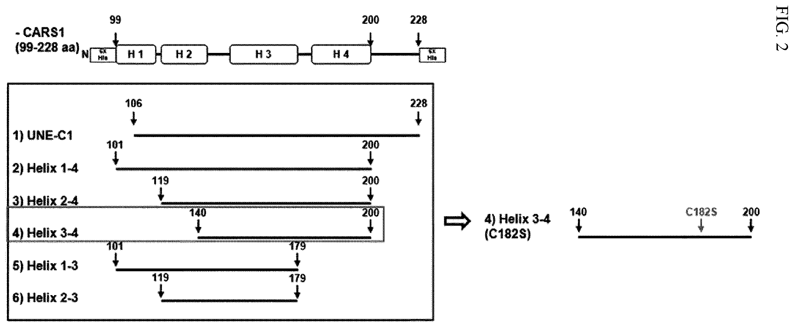

Based on the above experimental results, a gene was designed and cloned to substitute cysteine at position 182 with serine to inhibit multimer formation due to cysteine at position 182 in the fragment protein containing amino acids 140 to 200 of CRS. The protein was produced using BL21 DE3 codon plus RIPL cells for transformation (Fig. 2).

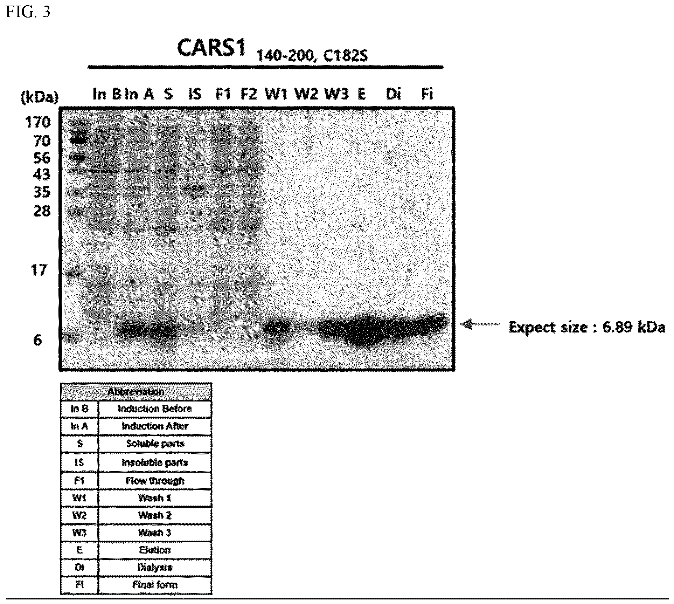

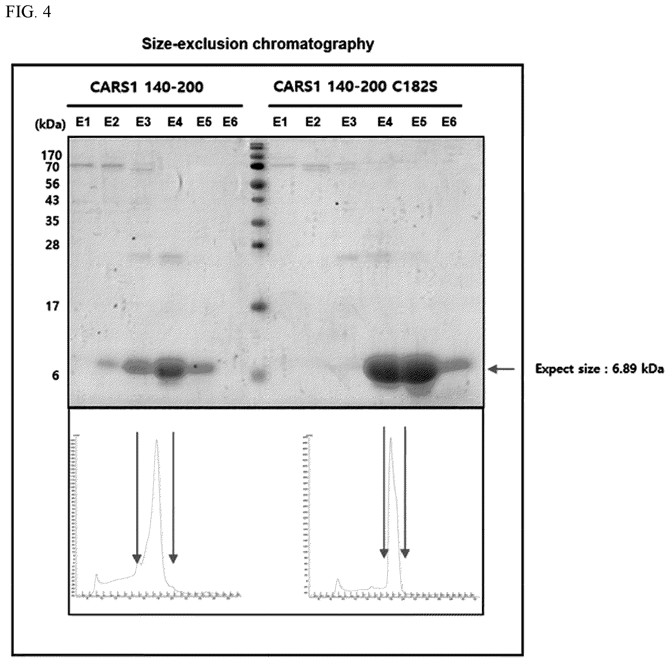

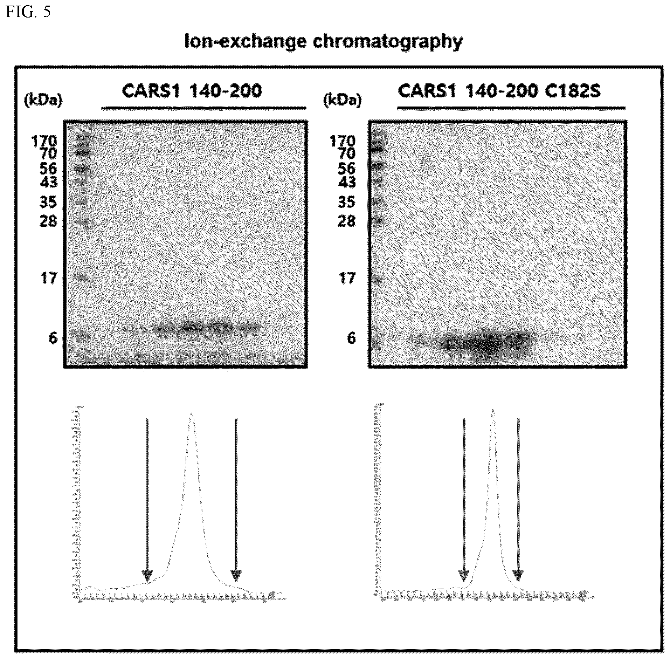

As a result, the degradation problem reported in previous studies was not observed (Fig. 3). It was also confirmed through gel filtration chromatography and ion exchange chromatography that the protein did not form multimers relatively compared to the protein without cysteine substitution at position 182 (Figs. 4 to 5).

### 3. Confirmation of Thermal Stability of CRS-Derived Fragment Protein CRS (140-200, C182S)

Based on the above experimental results, the thermal stability of the protein in which cysteine at position 182 was substituted with serine in the fragment protein containing amino acids 140 to 200 of CRS was confirmed through circular dichroism analysis and ELISA.

As a result, it was confirmed that the secondary structure was maintained at a level equivalent to the protein containing amino acids 106 to 228 of CRS, which was reported to have thermal stability in previous studies, compared to CRS-derived fragment proteins induced by high-temperature denaturation (Fig. 6A). Additionally, when the CRS-derived fragment protein induced by high-temperature denaturation was treated with Thp-1 cells differentiated with PMA and the amount of TNF-α in the medium was measured to observe immune activity, it was found that the function to induce immune activity was maintained despite high-temperature denaturation, indicating that the function was not lost at high temperatures due to thermal stability (Fig. 6B).

### 4. Confirmation of immune cell activation-inducing ability of CRS-derived fragment protein CRS (140-200, C182)

Based on the above experimental results, to confirm whether the immune cell activation-inducing ability of CRS-derived fragment protein CRS (140-200, C182S) is a protein-specific function, polymyxin b (10ug/ml), known as an LPS inhibitor, and proteinase K (20ug/ml), a protein-decomposing enzyme, were each treated, and then treated to Thp-1 differentiated with PMA.

As a result, it was observed that in the polymyxin b-treated group, the activity decreased in the case of LPS, but the activity of the CRS-derived fragment proteins did not decrease, which means that the induced immune activity is not due to LPS. And, in the case of the proteinase K treatment group, it was observed that the immune activity of the fragment proteins derived from CRS was reduced compared to LPS, which means that the induced immune activity is due to the protein. Therefore, the immune activity induction ability of the fragment protein CRS (140-200, C182S) derived from CRS is not due to LPS contamination, but is a function of the protein.

### 5. Confirmation of antiviral efficacy

### (1) Confirmation of antiviral efficacy by administration route and concentration of CARS1 (99-200, C182S) protein

The most significant changes caused by viral infections are known to be weight loss and decreased survival rates. To confirm antiviral efficacy, it is necessary to check how much the weight can recover to the pre-infection weight compared to the control group and whether survival is maintained over time. Additionally, since the invasion routes of each virus are different, the administration route of the candidate substance should also vary depending on the target virus.

The immune cell activation ability of CARS 1 induces intracellular reprogramming, which causes secondary stimulation and affects innate immune memory. This process is called trained immunity, which can regulate immune homeostasis and tolerance, and through this, it is reported that a defense mechanism against viruses can be established. Therefore, it was decided to confirm whether defense ability against viral infection is acquired through trained immunity induced by CARS 1-derived fragment proteins.

Accordingly, the present inventors confirmed the antiviral efficacy by injecting the CARS1 (99-200, C182S) protein at concentrations of 5, 10, and 20 mpk (mg/kg) using two administration routes, intraperitoneal and intranasal, to check the difference in efficacy according to the administration route. CARS1 (99-200, C182S) protein was administered to C57bl/6 mice on day-7 and day-3, and PR-8 influenza virus was administered intranasally on day-0 (Fig. 8A).

As a result, in the case of intraperitoneal administration, the survival rate increased at 10 and 20 mpk compared to saline, and the weight change was also maintained at a higher level at both concentrations (Fig. 8B to 8C). In the case of intranasal administration, the survival rate increased at 5, 10, and 20 mpk compared to saline, and the weight change was maintained at a higher level at all three concentrations compared to saline, with 10 mpk being the highest.

### (2) Confirmation of antiviral efficacy of CARS1 (99-200, C182S) protein compared to control group

According to the above results, Oseltamivir (Tamiflu), known as an influenza virus treatment, was used as a control group to compare the antiviral efficacy of CARS1 (99-200, C182S) protein. CARS1 (99-200, C182S) protein was administered to C57bl/6 mice on day-7 and day-3, and PR-8 influenza virus was administered intranasally on day-0. In the case of Oseltamivir, it was administered orally 4 hours before PR-8 influenza virus administration, and then the virus infection was proceeded (Fig. 9A).

As a result, the survival rate increased at 2 and 10 mpk of CARS1 (99-200, C182S) protein compared to saline, and at 10 mpk, the survival rate was maintained at a level similar to that of oseltamivir used as a control group (Fig. 9B).

### (3) Confirmation of antiviral efficacy of CARS1 (99-200, C182S) protein compared to control

### group against coronavirus

CARS1 (99-200, C182S) protein was administered to hACE-2 introduced C57bl/6 mice on day-7 and day-3, and SARS-Cov2 (S clade) virus was administered intranasally on day-0 (Fig. 10A).

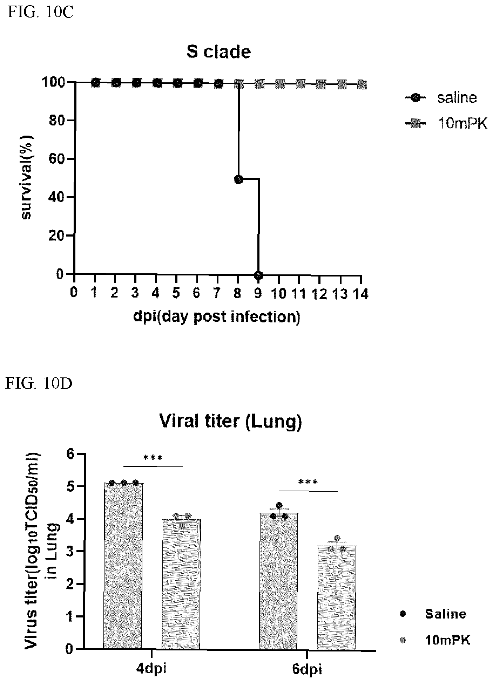

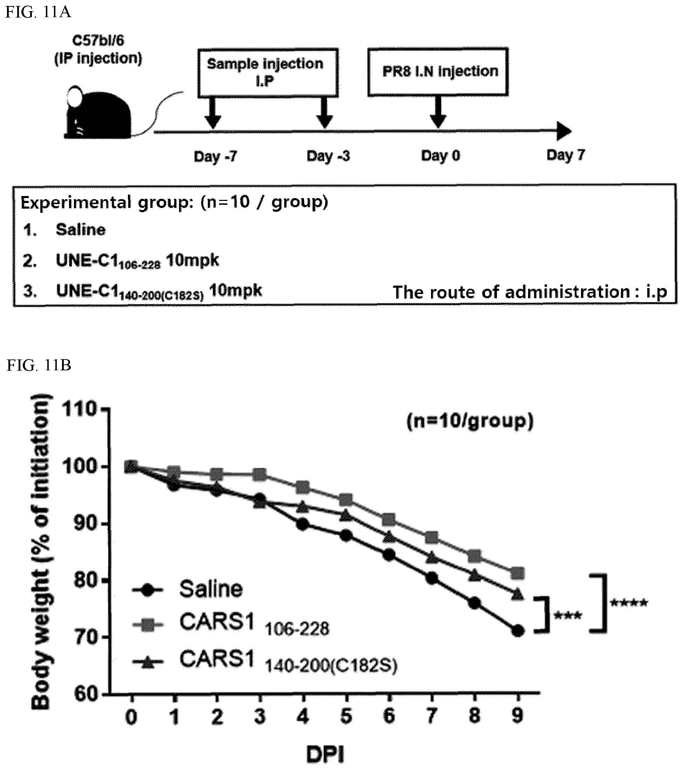

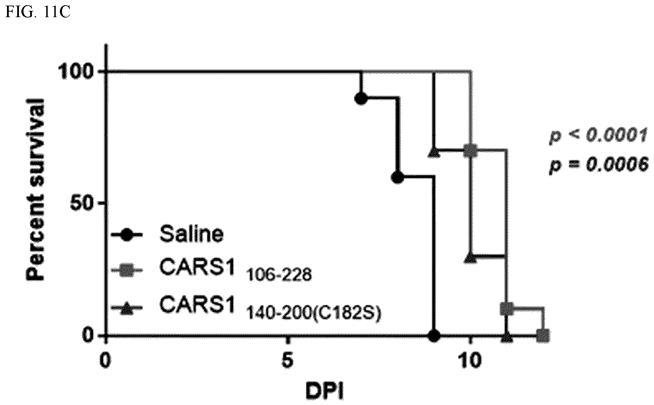

As a result, there was no weight loss at 10 mpk of CARS 1 (99-200, C182S) protein compared to saline (Fig. 10B), and all individuals survived (Fig. 10C). Additionally, when the animals were sacrificed on day 4 and day 6 and lung tissue was analyzed, the Cov-2 virus titer was significantly reduced in the CARS1 (99-200, C182S) protein-treated group compared to saline (Fig. 10D).

### (4) Confirmation of antiviral ability of CARS1 (140-200, C182S) protein

According to the above results, to compare the antiviral ability of the minimal unit form of CARS 1 (99-200, C182S) protein, which maintains the active part, with CARS1 (106-228), each protein was administered intraperitoneally at 10 mpk to C57bl/6 mice, and PR-8 influenza virus was administered intranasally on day-0, followed by checking weight change and survival rate (Fig. 10A).

As a result, weight gain was observed in both CARS1 (106-228) and CARS1 (140-200, C182S) proteins compared to saline (Fig. 10B), and the survival rate also increased in both proteins compared to saline (Fig. 10C).

## Claims

1. A pharmaceutical composition for antiviral use, comprising a peptide comprising consecutive amino acids sequence which is from any amino acid selected from 99th to 140th amino acids to any amino acid selected from 185th to 228th amino acids in the amino acid sequence of SEQ ID NO:1; a peptide comprising an amino acid sequence having 80% or more homology with the peptide; or a pharmaceutically acceptable salt thereof as an active ingredient.

2. The pharmaceutical composition of claim 1, wherein the peptide comprises a mutation in which the cysteine at position 182 in the amino acid sequence of SEQ ID NO: 1 is substituted with other amino acid.

3. The pharmaceutical composition of claim 2, wherein the other amino acid is serine.

4. The pharmaceutical composition of claim 1, wherein the peptide is selected from the group consisting of the amino acid sequences of SEQ ID NOs:2 to 8.

5. The pharmaceutical composition of claim 1, wherein the virus is selected from the group consisting of Amalgaviridae, Birnaviridae, Chrysoviridae, Cystoviridae, Endornaviridae, Hypoviridae, Megabirnaviridae, Partitiviridae, Picobirnaviridae, Reoviridae, Totiviridae, Quadriviridae, Arteriviridae, Coronaviridae, Mesoniviridae, Roniviridae, Dicistroviridae, Iflaviridae, Marnaviridae, Picornaviridae, Secoviridae, Alphaflexiviridae, Betaflexiviridae, Gammaflexiviridae, Tymoviridae, Bornaviridae, Filoviridae, Paramyxoviridae, Rhabdoviridae, Nyamiviridae, Caliciviridae, Flaviviridae, Luteoviridae, Togaviridae, Pneumoviridae, Arenaviridae, Deltavirus, and Orthomyxoviridae viruses.

6. The pharmaceutical composition of claim 1, wherein the virus is selected from the group consisting of influenza virus, influenza A virus subtype H1N1, avian influenza virus, rhinovirus, coronavirus, parainfluenza virus, respiratory syncytial virus, human immunodeficiency virus (HIV), retrovirus, and hepatitis C virus.

7. The pharmaceutical composition of claim 6, wherein the coronavirus is selected from the group consisting of severe acute respiratory syndrome coronavirus 2 (SARS-CoV-2), severe acute respiratory syndrome coronavirus (SARS-CoV), and Middle East respiratory syndrome (MERS) coronavirus.

8. A food composition for antiviral use, comprising a peptide comprising consecutive amino acids sequence which is from any amino acid selected from 99th to 140th amino acids to any amino acid selected from 185th to 228th amino acids in the amino acid sequence of SEQ ID NO: 1; a peptide comprising an amino acid sequence having 80% or more homology with the peptide; or a pharmaceutically acceptable salt thereof as an active ingredient.

9. The food composition of claim 8, wherein the food composition is a health functional food.

10. A quasi-drug composition for antiviral use, comprising a peptide comprising consecutive amino acids sequence which is from any amino acid selected from 99th to 140th amino acids to any amino acid selected from 185th to 228th amino acids in the amino acid sequence of SEQ ID NO:1; a peptide comprising an amino acid sequence having 80% or more homology with the peptide; or a pharmaceutically acceptable salt thereof as an active ingredient.

11. The quasi-drug composition of claim 10, wherein the quasi-drug is a sanitizing cleanser, nasal spray, shower foam, gargle, wet tissue, detergent soap, hand wash, humidifier filler, mask, ointment, patch, or filter filler.

12. An adjuvant composition for a viral vaccine, comprising a peptide comprising consecutive amino acids sequence which is from any amino acid selected from 99th to 140th amino acids to any amino acid selected from 185th to 228th amino acids in the amino acid sequence of SEQ ID NO:1; a peptide comprising an amino acid sequence having 80% or more homology with the peptide; or a pharmaceutically acceptable salt thereof as an active ingredient.

13. The adjuvant composition for a viral vaccine of claim 12, wherein the virus is selected from the group consisting of influenza virus, influenza A virus subtype H1N1, avian influenza virus, rhinovirus, coronavirus, parainfluenza virus, respiratory syncytial virus, human immunodeficiency virus (HIV), retrovirus, and hepatitis C virus.

14. The adjuvant composition for a viral vaccine of claim 12, wherein the coronavirus is selected from the group consisting of severe acute respiratory syndrome coronavirus 2 (SARS-CoV-2), severe acute respiratory syndrome coronavirus (SARS-CoV), and Middle East respiratory syndrome (MERS) coronavirus.

15. Use of a peptide comprising consecutive amino acids sequence which is from any amino acid selected from 99th to 140th amino acids to any amino acid selected from 185th to 228th amino acids in the amino acid sequence of SEQ ID NO:1; a peptide comprising an amino acid sequence having 80% or more homology with the peptide; or a pharmaceutically acceptable salt thereof for manufacturing a pharmaceutical composition for antiviral use.

16. A method for preventing or treating a viral infection, comprising administering to a subject in need thereof an effective amount of a composition comprising a peptide comprising consecutive amino acids sequence which is from any amino acid selected from 99th to 140th amino acids to any amino acid selected from 185th to 228th amino acids in the amino acid sequence of SEQ ID NO:1; or a peptide comprising an amino acid sequence having 80% or more homology with the peptide as an acitve ingredient.

17. A peptide consisting of the amino acid sequence of SEQ ID NO:2 or an amino acid sequence having 95% or more sequence homology thereto.

18. The peptide of claim 17, wherein the peptide comprises a mutation in which the cysteine at position 43 in the amino acid sequence of SEQ ID NO:2 is substituted with other amino acid.

19. The peptide of claim 18, wherein the other amino acid is serine.

20. A polynucleotide comprising a nucleotide sequence encoding the peptide of claim 17.

21. The polynucleotide of claim 20, wherein the polynucleotide consists of the nucleotide sequence of SEQ ID NO:9 or SEQ ID NO:10.

22. A vector comprising the polynucleotide of claim 20.

23. A host cell transformed with the vector of claim 22.

24. A vaccine adjuvant comprising one or more selected from the group consisting of:
(i) the peptide of claim 1,
(ii) a polynucleotide encoding (i),
(iii) a vector comprising (ii), and
(iv) a host cell transformed with (iii).

25. A vaccine composition comprising the vaccine adjuvant of claim 24 and an antigen.

26. The vaccine composition of claim 25, wherein the vaccine is an anticancer vaccine.

27. The vaccine composition of claim 25, wherein the anticancer vaccine is a vaccine for cancer prevention or a vaccine for cancer treatment.

28. A pharmaceutical composition for preventing or treating cancer, comprising one or more selected from the group consisting of:
(i) the peptide of claim 1,
(ii) a polynucleotide encoding (i),
(iii) a vector comprising (ii), and
(iv) a host cell transformed with (iii).
